# EUROPEAN PATENT SPECIFICATION

(11) **EP 0 336 228 B1**
(45) Date of publication and mention of the grant of the patent: **09.11.1994**
(21) Application number: 89105218.5
(22) Date of filing: 23.03.1989
(51) Int. Cl.: C07D 217/02, C07D 217/04, C07D 217/06, C07D 217/14, C07D 217/16, C07D 233/16, C07D 405/04, C07D 409/04, C07D 217/10, A61K 31/47

(54) **Tetrahydroisoquinolines and tetrahydrobenzazepines and a process for the preparation thereof**
Tetrahydroisochinoline und Tetrahydrobenzazepine und Verfahren zur Herstellung
Tétrahydroisoquinoléines et tétrahydrobenzazépines et procédé pour leur préparation

(30) Priority: 05.04.1988 GB 8807922
(43) Date of publication of application: 11.10.1989
(73) Proprietor: FUJISAWA PHARMACEUTICAL CO., LTD., Osaka-shi Osaka 541 (JP)
(72) Inventor: Takasugi, Hisashi, Osaka-shi Osaka 559 (JP); Kuno, Atsushi, Toyono-gun Osaka 563-01 (JP); Ohkubo, Mitsuru, Kawabe-gun Hyogo 666-02 (JP)
(74) Representative: Türk, Gille, Hrabal, Leifert

(56) References cited:
- EP-A- 0 230 871
- DE-A- 2 434 310
- Chemical Abstracts, vol. 90, no. 15, April 9, 1979, page 624, column 2, abstract-no. 121374s, Columbus, Ohio, US; H.W. Bersch et al.: "Syntheses and rearrangements of tertiary alpha-aryl-alkylamines"

## Description

The present invention relates to novel bicyclic amine compound and a pharmaceutically acceptable salt thereof.

More particularly, it relates to novel bicyclic amine compound and a pharmaceutically acceptable salt thereof, which is an N-methyl-D-aspartate (an excitatory amino acid) receptor antagonist and useful as an anticonvulsant and a drug for treatment of the delayed neuronal death induced, for instance, by cerebral ischemia, for example, in case of cardiac arrest, to a process for the preparation thereof, to a pharmaceutical composition comprising the same, and to a use of the same as a medicament in treatment of convulsion and delayed neuronal death in human being or animal.

Accordingly, one object of the present invention is to provide novel bicyclic amine compound and a pharmaceutically acceptable salt thereof, which is useful as stated above.

Another object of the present invention is to provide a process for the preparation of novel bicyclic amine compound and a salt thereof.

A further object of the present invention is to provide a pharmaceutical composition comprising, as an active ingredient, said bicyclic amine compound or a pharmaceutically acceptable salt thereof.

Still further object of the present invention is to provide a use of said bicyclic amine compound and a pharmaceutically acceptable salt thereof as a medicament in the treatment of convulsion and delayed neuronal death in human being or animal.

Some bicyclic amine compounds have been known as described, for example, in EP-A2-0 230 871, DE-A1-2 434 310 and CHEMICAL ABSTRACTS, vol. 90, page 624, abstract-no. 121374S.

The object bicyclic amine compound of the present invention is novel and can be represented by the following general formula (I). 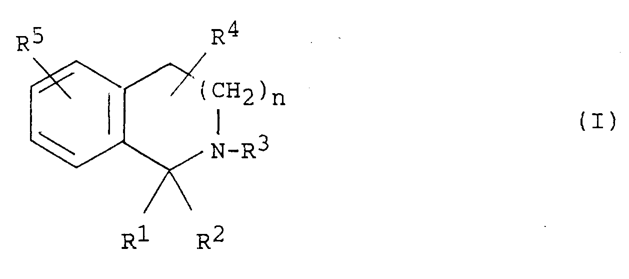
wherein
- R¹: is (C₁-C₆)alkyl,
- R²: is phenyl which may have 1 to 3 suitable substituent(s) selected from a group consisting of (C₁-C₆)alkyl, (C₁-C₆)alkoxy and halogen; cyclo(C₃-C₆)alkyl, furyl, or thienyl,
- R³: is hydrogen, (C₁-C₆)alkyl which may have 1 to 3 suitable substituent(s) selected from a group consisting of phenyl and piperidino, (C₂-C₆)alkenyl, or (C₁-C₆)alkanoyl which may have 1 to 3 suitable substituent(s) selected from a group consisting of halogen and piperidino,
- R⁴: is hydrogen, (C₁-C₆)alkyl, or hydroxy(C₁-C₆)alkyl,
- R⁵: is hydrogen, (C₁-C₆)alkyl, halogen, or (C₁-C₆)alkoxycarbonylamino, and
- n: is an integer of 1 or 2,
with proviso that R¹ is not ethyl, when R³ is methyl and n is 1.

According to the present invention, the novel bicyclic amine compound (I) can be prepared by the processes illustrated in the following reaction schemes. 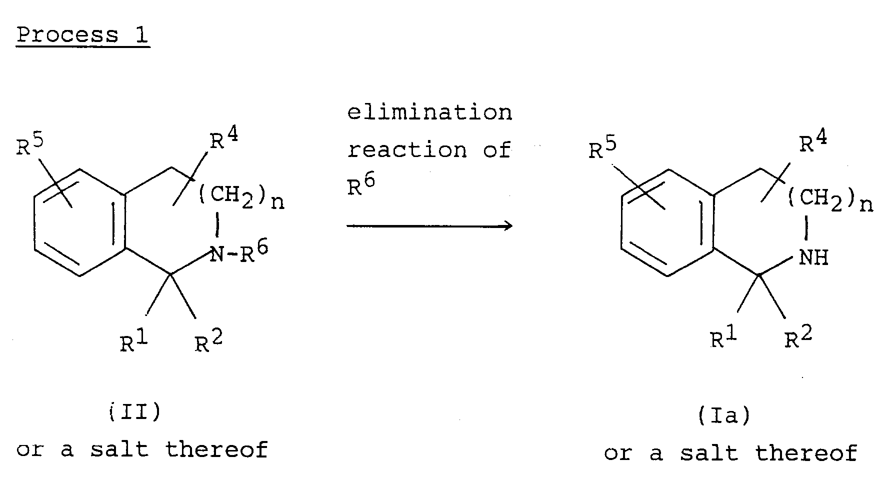 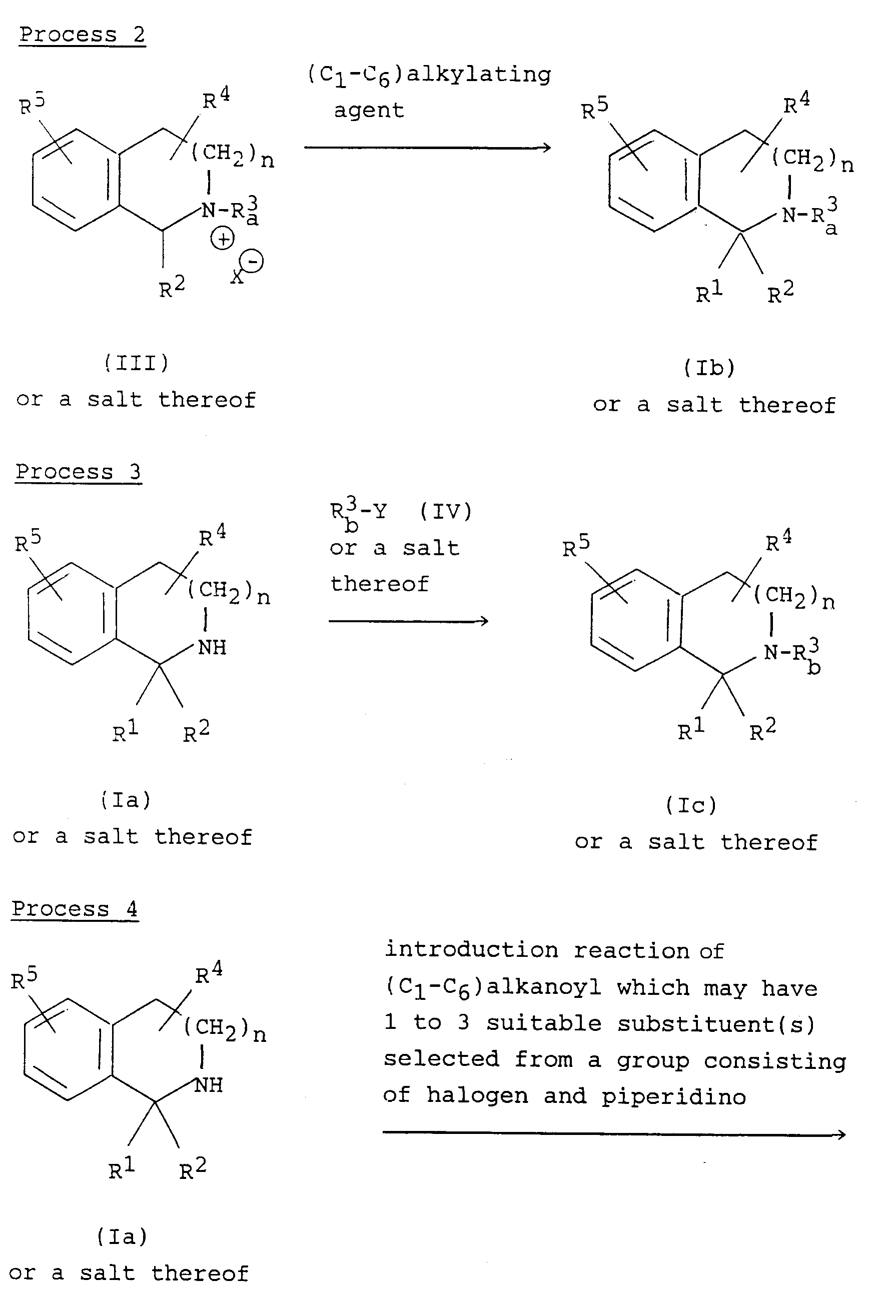 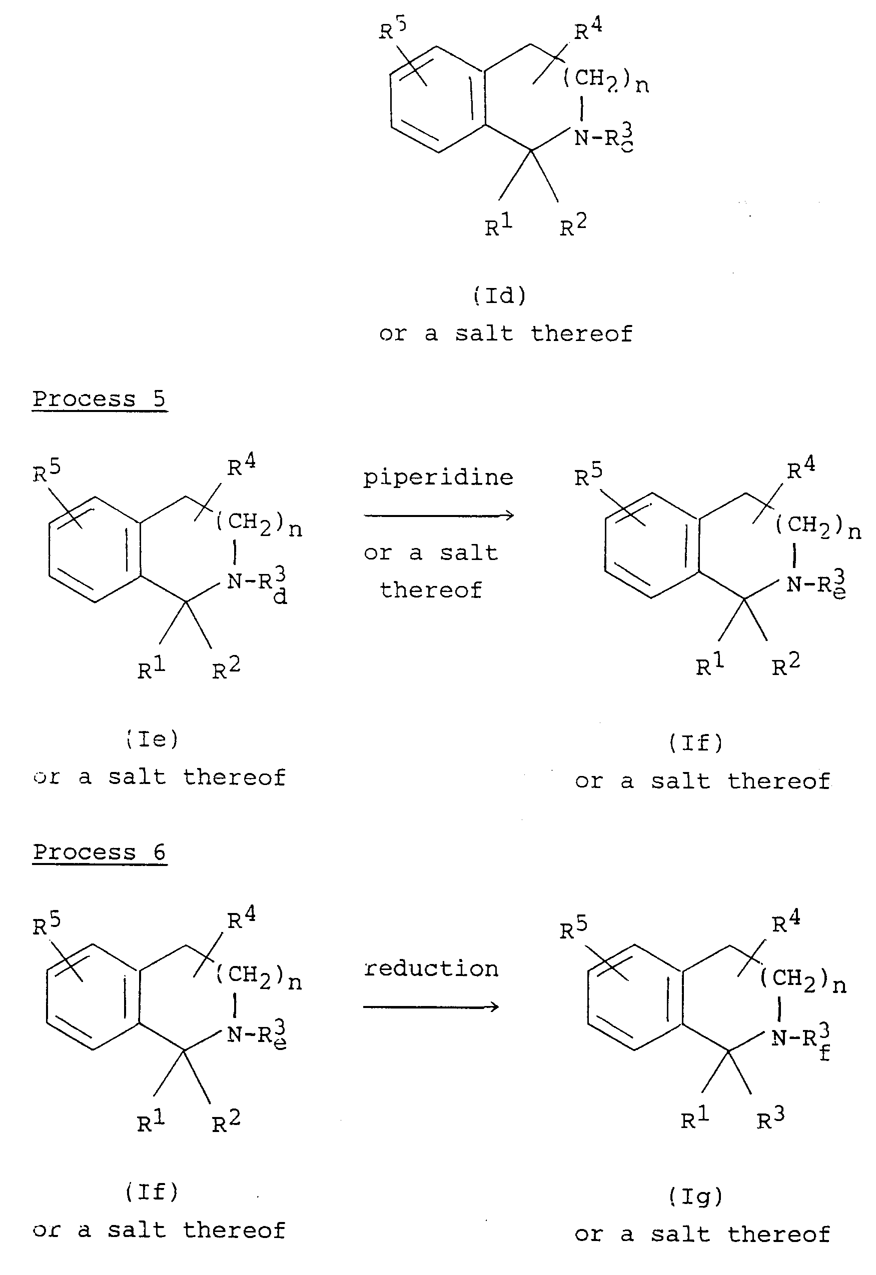
wherein
R¹, R², R⁴, R⁵ and n are each as defined above,
R$\frac{\text{3}}{\text{a}}$ is (C₁-C₆)alkyl which may have 1 to 3 suitable substituent(s) selected from a group consisting of phenyl and piperidino, (C₂-C₆)alkenyl, or (C₁-C₆)alkanoyl which may have 1 to 3 suitable substituent(s) selected from a group consisting of halogen and piperidino,
R$\frac{\text{3}}{\text{b}}$ is (C₁-C₆)alkyl which may have 1 to 3 suitable substituent(s) selected from a group consisting of phenyl and piperidino, or (C₂-C₆)alkenyl,
R$\frac{\text{3}}{\text{c}}$ is (C₁-C₆)alkanoyl which may have 1 to 3 suitable substituent(s) selected from a group consisting of halogen and piperidino,
R$\frac{\text{3}}{\text{d}}$ is (C₁-C₆)alkanoyl having halogen,
R$\frac{\text{3}}{\text{e}}$ is (C₁-C₆)alkanoyl having piperidino,
R$\frac{\text{3}}{\text{f}}$ is (C₁-C₆)alkyl having piperidino,
R⁶ is phenyl(C₁-C₆)alkyl which may have (C₁-C₆)alkoxy,
X^{⊖} is an anion, and
Y is a leaving group.

The starting compounds (II) and (III) to be used in these processes are novel and can be prepared by the processes illustrated in the following reaction schemes. 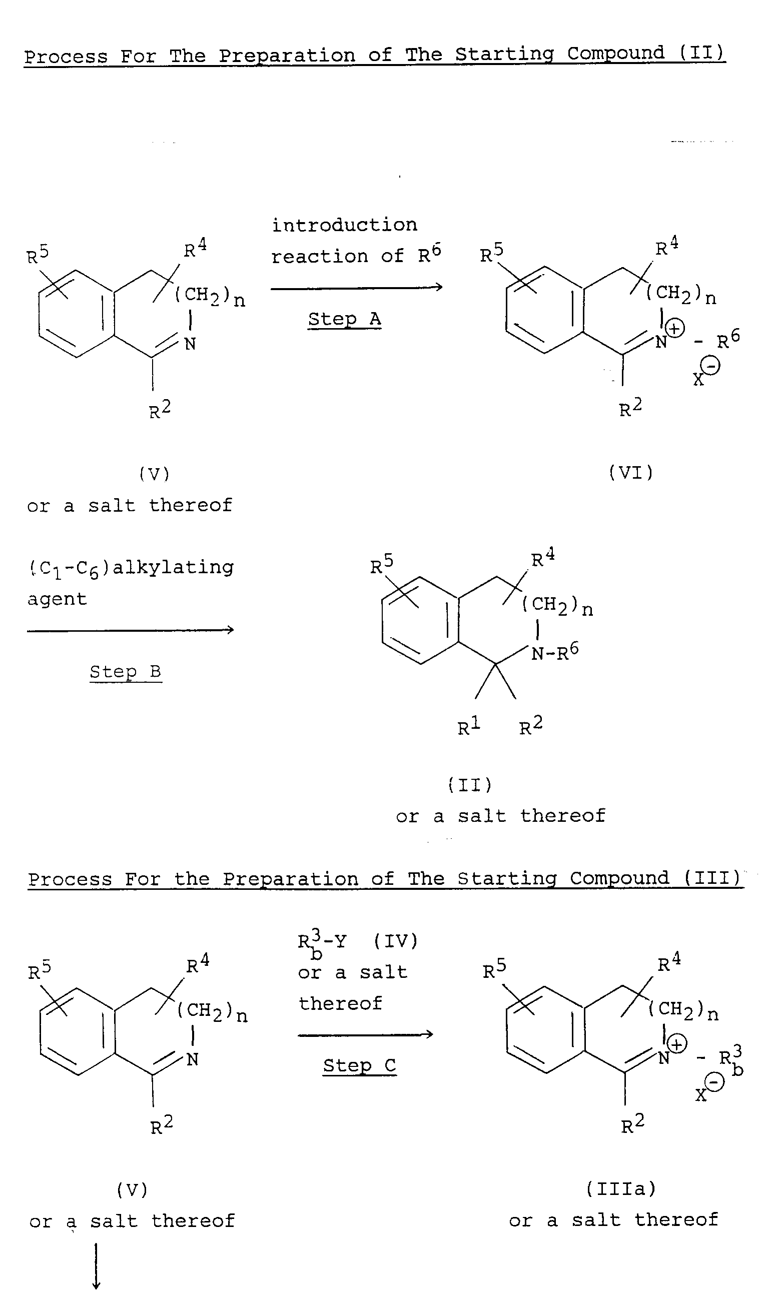 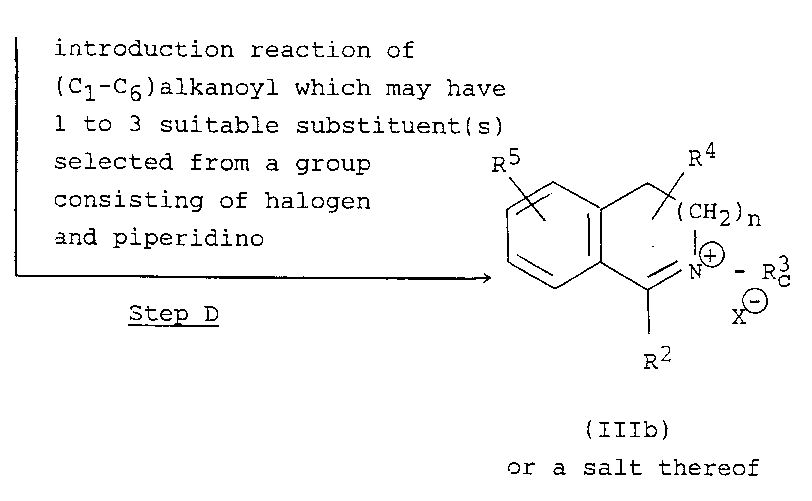
wherein
R¹, R², R$\frac{\text{3}}{\text{b}}$, R$\frac{\text{3}}{\text{c}}$, R⁴, R⁵, R⁶, n, X^{⊖} and Y are each as defined above.

The object compound (I), (Ia), (Ib), (Ic), (Id), (Ie), (If), (Ig) and the starting compound (II) include stereomers due to the existence of the asymmetric carbon atom therein. Said stereomers are also included within the scope of the present invention.

Suitable pharmaceutically acceptable salt of the object compound (I) is a conventional non-toxic salt and may include an acid addition salt such as an organic acid salt (e.g. acetate, trifluoroacetate, maleate, tartrate, methanesulfonate, benzenesulfonate, formate and toluenesulfonate), an inorganic acid salt (e.g. hydrochloride, hydrobromide, hydriodide, sulfate and phosphate) or a salt with an amino acid (e.g. arginine, aspartic acid and glutamic acid).

In the above and following descriptions of the present specification, suitable example and illustrations of the various definitions which the present invention include within the scope thereof are explained in detail as follows.

Suitable "(C₁-C₆)alkyl" may include straight or branched ones such as methyl, ethyl, propyl, isopropyl, butyl, tert-butyl, pentyl or hexyl, in which the preferred one may be (C₁-C₄)alkyl and the most preferred one may be methyl and ethyl.

The preferred "(C₁-C₆)alkyl which may have 1 to 3 suitable substituent(s) selected from a group consisting of phenyl and piperidino" may be (C₁-C₄)alkyl, (C₁-C₄)alkyl having phenyl and (C₁-C₄)alkyl having piperidino, and the more preferred one may be methyl, ethyl, phenethyl and 2-piperidinoethyl.

Suitable "(C₁-C₆)alkyl" in "phenyl which may have 1 to 3 suitable substituent(s) selected from a group consisting of (C₁-C₆)alkyl, (C₁-C₆)alkoxy and halogen" may include one as mentioned above, suitable "(C₁-C₆)alkoxy" in this group may include methoxy, ethoxy, propoxy, butoxy, t-butoxy, pentyloxy and hexyloxy, and suitable "halogen" in this group may include fluoro, chloro, bromo and iodo.

The preferred "phenyl which may have 1 to 3 suitable substituent(s) selected from a group consisting of (C₁-C₆)alkyl, (C₁-C₆)alkoxy and halogen" may be phenyl, phenyl having (C₁-C₆)alkyl (e.g. o-tolyl, m-tolyl, p-tolyl, 2-ethylphenyl, 3-propylphenyl, 4-butylphenyl, 3-t-butylphenyl, 4-pentylphenyl and 2-hexylphenyl), phenyl having (C₁-C₆)alkoxy (e.g. 2-methoxyphenyl, 4-methoxyphenyl, 3-ethoxyphenyl, 4-propoxyphenyl, 2-butoxyphenyl, 3-t-butoxyphenyl, 2-pentyloxyphenyl and 4-hexyloxyphenyl) and phenyl having halogen (e.g. 2-fluorophenyl, 3-fluorophenyl, 4-fluorophenyl, 2-chlorophenyl, 3-chlorophenyl, 4-chlorophenyl, 2-bromophenyl and 4-iodophenyl), and the more preferred one may be phenyl, p-tolyl, 4-methoxyphenyl, 3-fluorophenyl, 4-fluorophenyl, 2-chlorophenyl, 3-chlorophenyl and 4-chlorophenyl.

Suitable "cyclo(C₃-C₆)alkyl" may include cyclopropyl, cyclobutyl, cyclopentyl and cyclohexyl, in which the preferred one may be cyclo(C₄-C₆)alkyl and the more preferred one may be cyclohexyl.

Suitable "(C₂-C₆)alkenyl" may include straight or branched ones having 2 to 6 carbon atoms such as vinyl, allyl, isopropenyl, 2-butenyl, 4-pentenyl or 1-hexenyl, in which the preferred one may be (C₂-C₄)alkenyl and the most preferred one may be allyl.

Suitable "(C₁-C₆)alkanoyl" may include formyl, acetyl, propionyl, butyryl, valeryl, pivaloyl and hexanoyl, and said "(C₁-C₆)alkanoyl" may have 1 to 3 suitable substituent(s) such as aforesaid halogen or piperidino.

The preferred "(C₁-C₆)alkanoyl which may have 1 to 3 suitable substituent(s) selected from a group consisting of halogen and piperidino" may be (C₁-C₆)alkanoyl, (C₁-C₆)alkanoyl having halogen, (C₁-C₆)alkanoyl having piperidino, the more preferred one may be (C₁-C₆)alkanoyl having halogen and (C₁-C₆)alkanoyl having piperidino, the much more preferred one may be (C₂-C₄)alkanoyl having halogen and (C₂-C₄)alkanoyl having piperidino, and the most preferred one may be 2-chloroacetyl and 2-piperidinoacetyl.

Suitable "hydroxy(C₁-C₆)alkyl" may include hydroxymethyl, 2-hydroxyethyl, 2-hydroxypropyl, 1-hydroxybutyl, 1-hydroxymethyl-1-methylethyl, 3-hydroxypentyl, 6-hydroxyhexyl, in which the preferred one may be hydroxy(C₁-C₄)alkyl and the more preferred one may be hydroxymethyl.

Suitable "(C₁-C₆)alkoxy" in "phenyl(C₁-C₆)alkyl which may have (C₁-C₆)alkoxy" may be aforesaid (C₁-C₆)alkoxy and the preferred "phenyl(C₁-C₆)alkyl which may have (C₁-C₆)alkoxy" may be phenyl(C₁-C₄)alkyl which may have (C₁-C₄)alkoxy and the more preferred one may be benzyl and 4-methoxybenzyl.

Suitable "anion" may be formate, acetate, trifluoroacetate, maleate, tartrate, methanesulfonate, benzenesulfonate, toluenesulfonate, halogen anion (e.g. chloride, bromide, iodide), sulfate or phosphate.

Suitable "a leaving group" may include halogen as mentioned before, and acyloxy such as (C₁-C₆)alkanoyloxy (e.g. acetoxy) or sulfonyloxy (e.g. mesyloxy and tosyloxy).

The precesses for preparing the object compound (I) of the present invention are explained in detail in the following.

### Process 1

The compound (Ia) or a salt thereof can be prepared by subjecting the compound (II) or a salt thereof to elimination reaction of R⁶.

Suitable salts of the compounds (Ia) and (II) can be referred to the ones as exemplified for the compound (I).

This reaction is carried out in accordance with a conventional method such as hydrolysis, reduction or oxidation.

The hydrolysis is preferably carried out in the presence of a base or an acid including Lewis acid. Suitable base may include an inorganic base and an organic base such as an alkali metal [e.g. sodium and potassium], an alkaline earth metal [e.g. magnesium and calcium], the hydroxide or carbonate or bicarbonate thereof, trialkylamine [e.g. trimethylamine and triethylamine], picoline, 1,5-diazabicyclo[4.3.0]non-5-ene, 1,4-diazabicyclo[2.2.2]octane or 1,8-diazabicyclo[5.4.0]undec-7-ene.

Suitable acid may include an organic acid [e.g. formic acid, acetic acid, propionic acid, trichloroacetic acid and trifluoroacetic acid] and an inorganic acid [e.g. hydrochloric acid, hydrobromic acid, sulfuric acid, hydrogen chloride and hydrogen bromide]. The elimination using Lewis acid such as trihaloacetic acid [e.g. trichloroacetic acid and trifluoroacetic acid] is preferably carried out in the presence of cation trapping agents [e.g. anisole and phenol].

The reaction is usually carried out in a solvent such as water, an alcohol [e.g. methanol and ethanol], methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.

The reduction method applicable for the elimination reaction may include chemical reduction and catalytic reduction.

Suitable reducing agents to be used in chemical reduction are a combination of metal [e.g. tin, zing and iron] or metallic compound [e.g. chromium chloride and chromium acetate] and an organic or inorganic acid [e.g. formic acid, acetic acid, propionic acid, trifluoroacetic acid, p-toluenesulfonic acid, hydrochloric acid and hydrobromic acid].

Suitable catalysts to be used in catalytic reduction are conventional ones such as platinum catalysts [e.g. platinum plate, spongy platinum, platinum black, colloidal platinum, platinum oxide and platinum wire], palladium catalysts [e.g. spongy palladium, palladium black, palladium oxide, palladium on carbon, colloidal palladium, palladium on barium sulfate and palladium on barium carbonate], nickel catalysts [e.g. reduced nickel, nickel oxide and Raney nickel], cobalt catalysts [e.g. reduced cobalt and Raney cobalt], iron catalysts [e.g. reduced iron and Raney iron] or copper catalysts [e.g. reduced copper, Raney copper and Ullman copper].

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran or acetic acid, or a mixture thereof.

The reaction temperature of this reduction is not critical and the reaction is usually carried out under cooling to warming.

The oxidation method applicable for the elimination reaction may include oxidation using an oxidating agent such as cerium compound (e.g. ceric ammonium nitrate).

The oxidation is usually carried out in a conventional solvent such as water, alcohol (e.g. methanol and ethanol), acetonitrile or any other solvent which does not adversely influence the reaction.

The reaction temperature of this oxidation is not critical and the reaction is usually carried out under cooling to warming.

### Process 2

The compound (Ib) or a salt thereof can be prepared by reacting the compound (III) of a salt thereof with a (C₁-C₆)alkylating agent.

Suitable salts of the compounds (Ib) and (III) can be referred to the ones as exemplified for the compound (I).

Suitable examples for said (C₁-C₆)alkylating agent may be so-called Grignard type reagent shown in the formula R¹MgY (wherein R¹ is (C₁-C₆)alkyl and Y is halogen, each explained before) (e.g. methylmagnesium bromide, ethylmagnesium bromide and propylmagnesium iodide), or metalated (C₁-C₆)alkyl (e.g. methyl lithium and ethyl lithium).

The reaction is usually carried out in a solvent such as diethyl ether, tetrahydrofuran or any other solvent which does not adversely affect the reaction.

The reaction temperature is not critical and usually carried out under cooling to heating.

### Process 3

The compound (Ic) or a salt thereof can be prepared by reacting the compound (Ia) or a salt thereof with the compound (IV) or a salt thereof.

Suitable salts of the compounds (Ic) and (IV) can be referred to the ones as exemplified for the compound (I).

The present reaction may be carried out in a solvent such as water, phosphate buffer, acetone, chloroform, acetonitrile, nitrobenzene, methylene chloride, ethylene chloride, formamide, N,N-dimethylformamide, methanol, ethanol, diethyl ether, tetrahydrofuran, dimethyl sulfoxide, or any other organic solvent which does not adversely affect the reaction, preferably in ones having strong polarities. Among the solvents, hydrophilic solvents may be used in a mixture with water. When the compound (IV) is in liquid, it can also be used as a solvent.

The reaction is preferably conducted in the presence of a base, for example, inorganic base such as alkali metal hydride (e.g. sodium hydride), alkali metal hydroxide, alkali metal carbonate, alkali metal bicarbonate, or organic base such as trialkylamine.

The reaction temperature is not critical, and the reaction is usually carried out at room temperature, under warming or under heating.

The present reaction is preferably carried out in the presence of alkali metal halide [e.g. sodium iodide and potassium iodide] or alkali metal thiocyanate [e.g. sodium thiocyanate and potassium thiocyanate].

### Process 4

The compound (Id) or a salt thereof can be prepared by subjecting the compound (Ia) or a salt thereof to introduction reaction of (C₁-C₆)alkanoyl which may have 1 to 3 suitable substituent(s) selected from the group consisting of halogen and piperidino.

Suitable salt of the compound (Id) can be referred to the ones as exemplified for the compound (I).

This introduction reaction can be carried out by reacting the compound (Ia) or a salt thereof with an acid corresponding to (C₁-C₆)alkanoyl to be introduced or its reactive derivative at the carboxy group or a salt thereof.

Suitable reactive derivative at the carboxy group may include an acid halide (e.g. acid chloride), an acid anhydride, an activated amide and an activated ester, which are used conventionally in this field of the art.

This reaction is usually carried out in a conventional solvent such as methylene chloride or chloroform.

The reaction temperature is not critical and the reaction can be carried out under cooling to warming.

### Process 5

The compound (If) or a salt thereof can be prepared by reacting the compound (Ie) or a salt thereof with piperidine or a salt thereof.

Suitable salts of the compounds (Ie) and (If), and piperidine can be referred to the ones as exemplified for the compound (I).

This reaction can be carried out according to a similar manner to that of explained in Process 3.

### Process 6

The compound (Ig) or a salt thereof can be prepared by subjecting the compound (If) or a salt thereof to reaction.

Suitable salt of the compound (Ig) can be referred to the ones as exemplified for the compound (I).

Suitable reducing agent for this reduction may include lithium aluminum hydride and sodium borohydride.

This reaction is usually carried out in a conventional solvent such as tetrahydrofuran or diethyl ether.

The reaction temperature is not critical and the reaction can be carried out under cooling to heating.

When the compound (I) thus obtained in these processes is a racemic mixture, this mixture can be separated into each stereomer according to a conventional manner.

The processes for preparing the starting compounds (II) and (III) of the present invention are explained in detail in the following.

### Step A

The compound (VI) can be prepared by subjecting the compound (V) or a salt thereof to introduction reaction of R⁶.

Suitable salt of the compound (V) can be referred to the ones as exemplified for the compound (I).

The introduction reaction of R⁶ in this step can be carried out by reacting the compound (V) or a salt thereof with a suitable agent for introducing R⁶.

Suitable examples of said agent may be phenyl(C₁-C₆)alkyl halide which may have aforesaid (C₁-C₆)alkoxy (e.g. benzyl iodide, 3-methoxybenzyl iodide, benzyl bromide, 4-methoxybenzyl bromide and phenethyl chloride).

This introduction reaction may be carried out in a suitable solvent such as chloroform, acetonitrile, nitrobenzene, N,N-dimethylformamide or any other solvent which does not adversely affect the reaction.

The reaction temperature is not critical and usually carried out at room temperature, under warming or under heating.

### Step B

The compound (II) or a salt thereof can be prepared by reacting the compound (VI) with a (C₁-C₆)alkylating agent.

This reaction can be carried out according to a similar manner to that as explained in Process 2.

### Step C

The compound (IIIa) or a salt thereof can be prepared by reacting the compound (V) or a salt thereof with the compound (IV) or a alt thereof.

Suitable salt of the compound (IIIa) can be referred to the ones as exemplified for the compound (I).

This reaction can be carried out according to a similar manner to that as explained in Process 3.

### Step D

The compound (IIIb) or a salt thereof can be prepared by subjecting the compound (V) or a salt thereof to introduction reaction of (C₁-C₆)alkanoyl which may have 1 to 3 suitable substituent(s) selected from a group consisting of halogen and piperidino.

Suitable salt of the compound (IIIb) can be referred to the ones as exemplified for the compound (I).

This reaction can be carried out according to a similar manner to that as explained in Process 4.

The object bicyclic amine compound (I) of the present invention is an N-methyl-D-aspartate receptor antagonist and useful as an anticonvulsant and a drug for treatment of the delayed neuronal death induced, for instance, by cerebral ischemia, for example, in case of cardiac arrest in myocardial infarction.

In order to show the usefulness of the object compound (I), we set forth the representative test results in the following.

### Test 1 : the effect against the convulsion

### [I] Test Compound

1-Methyl-1-phenyl-1,2,3,4-tetrahydroisoquinoline hydrochloride (the compound of Example 1)

### [II] Test Method

Six- to seven-week-old ICR male mice (5 mice per one group) were used for this experiment.

Test compound dissolved in saline was administered intraperitoneally (dose : 100 mg/kg) to each mouse (Test Group).

To the Control Group, only saline was administered. 30 minutes after said administration, N-methyl-D-aspartate (0.32 µg) was injected intracerebroventricularly to each mouse. Then, each mouse was put into a plastic cage and observed for 10 minutes to confirm the occurrence of clonic and tonic seizures.

### [III] Test Results

The latency of initial seizure (second) (mean value ± standard error) was shown in the following table.

| | Latency (second) |
|---|---|
| Control Group | 0.5 ± 0.7 |
| Test Group | > 600** |

| | |
|---|---|
| ** P < 0.01 | |

### Test 2 : the effect against the convulsion

### [I] Test Compound

(+)-1-Methyl-1-phenyl-1,2,3,4-tetrahydroisoquinoline hydrochloride (the compound of Example 4)

### [II] Test Method

the same as used in Test 1

### [III] Test Results

The latency of initial seizure (second) (mean value ± standard error) was shown in the following table.

| | Latency (second) |
|---|---|
| Control Group | 8.4 ± 0.5 |
| Test Group | > 600** |

| | |
|---|---|
| ** P < 0.01 | |

### Test 3 : the effect against the delayed neuronal death

### [I] Test Compound

1-Methyl-1-phenyl-1,2,3,4-tetrahydroisoquinoline hydrochloride (the compound of Example 1)

### [II] Test Method

The effect on ischemia-induced delayed hippocampal neurodegeneration was examined according to the following method :
Eight- to nine-week-old male Wistar rats were used for this experiment.

The vertebral arteries were cauterized within the alar foramina and the common carotid arteries were exposed and looped with surgical suture under anesthesia with thiopental sodium (50 mg/kg i.p.).

Next day, the both carotid arteries were occluded with aneurysm clips for 20 minutes under a slight ether anesthesia. The test compound suspended in 0.5% methylcellulose (dose : 100 mg/kg) was administered intraperitoneally 10 minutes prior to ischemic insult (Test Group). To control Group, only 0.5% methylcellulose was administered.

Seven days after the recirculation, rate were perfusion-fixed with fixative consisting of 1.5% glutamic dialdehyde and 1.0% paraformaldehyde in 0.1M phosphate buffer (pH = 7.4). Perfusion was performed at a pressure of 160 cm H₂O.

Neuronal cell damage was assessed by counting the number of pyramidal neurons appearing normal in a 1 mm length of CA 1 pyramidal cell layer from each hippocampus in coronal sections (3 ∼ 4 µm) stained by cresyl violet corresponding to 1.9-2.1 mm posterior to the bregma.

### [III] Test Results

The number of normal pyramidal neurons in CA 1 (cells/mm) (mean value ± standard error) in each group was shown in the following table.

| group | the number of rats to be used | the number of normal pyramidal neurons in CA 1 |
|---|---|---|
| Normal Group | 8 | 136.0 ± 3.1 |
| Control Group | 7 | 20.9 ± 13.4 |
| Test Group | 4 | 88.3 ± 27.5* |

| | | |
|---|---|---|
| * P < 0.05 : compared with the control group | | |

### Test 4 : the effect against the delayed neuronal death

### [I] Test Compound

(+)-1-Methyl-1-phenyl-1,2,3,4-tetrahydroisoquinoline hydrochloride (the compound of Example 4)

### [II] Test Method

The effect on ischemia-induced delayed hippocampal neurodegeneration was examined according to the following method :
Eight- to nine-week-old male Wistar rats were used for this experiment.

The vertebral arteries were cauterized within the alar foramina and the common carotid arteries were exposed and looped with surgical suture under anesthesia with thiopental sodium (50 mg/kg i.p.).

Next day, the both carotid arteries were occluded with aneurysm clips for 20 minutes under a slight ether anesthesia. The test compound suspended in 0.5% methylcellulose (dose : 32 mg/kg) was administered intraperitoneally 10 minutes prior to ischemic insult (Test Group). To Control Group, only 0.5% methylcellulose was administered.

Seven days after the recirculation, rats were perfusion-fixed with fixative consisting of 1.5% glutamic dialdehyde and 1.0% paraformaldehyde in 0.1M phosphate buffer (pH = 7.4). Perfusion was performed at a pressure of 160 cm H₂O.

Neuronal cell damage was assessed by counting the number of pyramidal neurons appearing normal in a 1 mm length of CA 1 pyramidal cell layer from each hippocampus in coronal sections (3 ∼ 4 µm) stained by cresyl violet corresponding to 1.9-2.1 mm posterior to the bregma.

### [III] Test Results

The number of normal pyramidal neurons in CA 1 (cells/mm) (mean value ± standard error) in each group was shown in the following table.

| group | the number of rats to be used | the number of normal pyramidal neurons in CA 1 |
|---|---|---|
| Normal Group | 8 | 136.0 ± 3.1 |
| Control Group | 7 | 20.9 ± 13.4 |
| Test Group | 5 | 107.2 ± 16.9** |

| | | |
|---|---|---|
| ** P < 0.01 : compared with the control group | | |

### Test 5 : the effect against the delayed neuronal death

### [I] Test Compound

(+)-1-Methyl-1-phenyl-1,2,3,4-tetrahydroisoquinoline hydrochloride (the compound of Example 4)

### [II] Test Method

The effect on ischemia-induced delayed hippocampal neurodegeneration was examined according to the following method :
Eight- to ten-week-old male gerbils were used for this experiment.

The both common carotid arteries were exposed under local anesthesia with lidocaine and were occluded with aneurysm clips for 5 minutes. The test compound dissolved in saline (dose : 10 mg/kg) was administered intraperitoneally 30 minutes prior to ischemic insult (Test Group). To Control Group, only saline was administered.

Four days after the recirculation, gerbils were perfusion-fixed with fixative consisting of 1.5% glutamic dialdehyde and 1.0% paraformaldehyde in 0.1M phosphate buffer (pH = 7.4). Perfusion was performed at a pressure of 160 cm H₂O.

Neuronal cell damage was assessed by counting the number of pyramidal neurons appearing normal in a 1 mm length of CA 1 pyramidal cell layer from each hippocampus in coronal sections (3 ∼ 4 µm) stained by cresyl violet corresponding to 0.5 - 1 mm posterior to the bregma.

### [III] Test Results

The number of normal pyramidal neurons in CA 1 (cells/mm) (mean value ± standard error) in each group was shown in the following table.

| group | the number of gerbils to be used | the number of normal pyramidal neurons in CA 1 |
|---|---|---|
| Normal Group | 7 | 201.0 ± 6.5** |
| Control Group | 7 | 3.9 ± 0.9 |
| Test Group | 7 | 91.9 ± 30.2** |

| | | |
|---|---|---|
| ** P < 0.01 : compared with the control group | | |

For therapeutic administration, the object compound (I) and pharmaceutically acceptable salts thereof of the present invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration. The pharmaceutical preparations may be in solid form such as tablet, granule, powder, capsule, or liquid form such as solution, suspension, syrup, emulsion or lemonade.

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter or ethylene glycol.

While the dosage of the compound (I) may vary from and also depend upon the age, conditions of the patient, a kind of diseases or a kind of the compound (I) to be applied. In general, amounts between 1 mg and about 6,000 mg or even more per day may be administered to a patient. An average single dose of about 50 mg, 100 mg, 250 mg, 500 mg, 1000 mg, 2000 mg of the object compound (I) of the present invention may be used.

The following Preparations and Examples are given for the purpose of illustrating the present invention in more detail.

### Preparation 1

A mixture of 1-phenyl-3,4-dihydroisoquinoline (1.0 g) and benzyliodide (1.26 g) in acetonitrile (7 ml) was refluxed for 1 hour. After allowing to cool to room temperature, the reaction mixture was evaporated in vacuo and the residual precipitate was recrystallized from a mixture of diethyl ether (5 ml) and acetonitrile (3 ml). The crystal was collected by filtration, washed with diethyl ether and dried in vacuo to give 1-phenyl-2-benzyl-3,4-dihydroisoquinolinium iodide (1.52 g).
mp : 208-209°C
IR (Nujol) : 1620, 1600, 1565 cm⁻¹
NMR (DMSO-d₆, δ) : 3.52 (2H, t, J=7Hz), 4.20 (2H, t, J=7Hz), 5.10 (2H, s), 6.96-7.83 (14H, m)

### Preparation 2

The following compound was obtained according to a similar manner to that of Preparation 1.

l-(3-Chlorophenyl)-2-benzyl-3,4-dihydroisoquinolinium iodide
mp : l9l-l92°C
IR (Nujol) : l6l5, l595, l560 cm⁻¹
NMR (CDCl₃, δ) : 3.33 (2H, t, J=7Hz), 4.25 (2H, t, J=7Hz), 5.l2 (2H, s), 7.06-8.02 (l4H, m)

### Preparation 3

To a suspension of l-phenyl-2-benzyl-3,4-dihydroisoquinolinium iodide (l.0 g) in diethyl ether (l0 ml) was added 3 M diethyl ether solution of methylmagnesium bromide (4.5 ml). The mixture was refluxed for 30 minutes with stirring and then poured into saturated ammonium chloride aqueous solution and extracted with ethyl acetate. The separated organic layer was washed with water, sodium chloride aqueous solution and dried over magnesium sulfate and evaporated in vacuo. The residue was crystallized from n-hexane to give l-methyl-l-phenyl-2-benzyl-l,2,3,4-tetrahydroisoquinoline (0.64 g).
mp : l02-l03°C
IR (Nujol) : l600, l580, l485 cm⁻¹
NMR (CDCl₃, δ) : l.78 (3H, s), 2.58-3.l0 (4H, m), 3.22 (lH, d, J=l4Hz), 3.56 (lH, d, J=l4Hz), 6.60-7.65 (l4H, m)
Mass : 3l3 (M⁺)

The following compounds (Preparations 4 and 5) were obtained according to a similar manner to that of Preparation 3.

### Preparation 4

l-Ethyl-l-phenyl-2-benzyl-l,2,3,4-tetrahydroisoquinoline
mp : 96-97°C (recrystallized from n-hexane)
IR (Nujol) : l600, l490 cm⁻¹
NMR (CDCl₃, δ) : 0.85 (3H, t, J=7Hz), 2.l2 (lH, dd, J=7Hz and l4Hz), 2.46-3.05 (5H, m), 3.88 (lH, d, J=l4Hz), 6.70-7.30 (l4H, m)
Mass : 327 (M⁺)

### Preparation 5

l-Methyl-l-(3-chlorophenyl)-2-benzyl-l,2,3,4-tetrahydroisoquinoline
NMR (CDCl₃, δ) : l.8l (3H, s), 2.78-3.75 (6H, m), 6.62-7.70 (l4H, m)
Mass : 347 (M⁺)

### Example 1

A mixture of l-methyl-l-phenyl-2-benzyl-l,2,3,4-tetrahydroisoquinoline (0.5 g) and l0% palladium on carbon (50% wet 0.l g) in acetic acid (l5 ml) was hydrogenated under one atmospheric pressure of hydrogen at room temperature for 9 hours. Insoluble material was filtered off and the filtrate was concentrated in vacuo. The residue was dissolved in ethyl acetate and washed with saturated sodium bicarbonate aqueous solution, water, sodium chloride aqueous solution and dried over magnesium sulfate and evaporated in vacuo. The residue was dissolved in ethanol and to the solution was added 7 M ethanol solution of hydrogen chloride. The precipitate was collected by filtration, washed with ethanol and diethyl ether and dried in vacuo to give l-methyl-l-phenyl-l,2,3,4-tetrahydroisoquinoline hydrochloride (0.4 g).
mp : 274-276°C (decomp.)
IR (Nujol) : l585, l490 cm⁻¹
NMR (DMSO-d₆, δ) : 2.l0 (3H, s), 2.80-3.50 (4H, m), 7.05-7.45 (9H, m), 9.80 (lH, s), l0.40 (lH, s)
Mass : 222 (M⁺-l)

| Elemental Analysis for C₁₆H₁₇N^{.}HCl | | | |
|---|---|---|---|
| Calcd. | C 73.98, | H 6.98, | N 5.45 |
| Found | C 73.55, | H 6.98, | N 5.28 |

The following compounds (Examples 2 and 3) were obtained according to a similar manner to that of Example 1.

### Example 2

l-Ethyl-l-phenyl-l,2,3,4-tetrahydroisoquinoline hydrochloride
mp : 288-290°C
IR (Nujol) : l580, l495 cm⁻¹
NMR (DMSO-d₆, δ) : 0.98 (3H, t, J=7Hz), 2.56-3.36 (6H, m), 7.l6-7.36 (9H, m), 9.65-l0.26 (2H, broad)
Mass : 236 (M⁺-l)

| Elemental Analysis | | | |
|---|---|---|---|
| Calcd. | C 74.58, | H 7.36, | N 5.l2 |
| Found | C 74.57, | H 7.42, | N 5.08 |

### Example 3

l-Methyl-l-(3-chlorophenyl)-l,2,3,4-tetrahydroisoquinoline hydrochloride
mp : 265-267°C
IR (Nujol) : l580, l495 cm⁻¹
NMR (DMSO-d₆, δ) : 2.l3 (3H, s), 2.90-3.55 (4H, m), 7.05-7.50 (8H, m)
Mass : 257 (M⁺)

| Elemental Analysis | | | |
|---|---|---|---|
| Calcd. | C 65.32, | H 5.82, | N 4.76 |
| Found | C 65.l0, | H 5.65, | N 4.78 |

### Example 4

(l) l-Methyl-l-phenyl-l,2,3,4-tetrahydroisoquinoline (racemic mixture) (l0.2 g) and (+)-di-p-toluoyl-D-tartaric acid (l7.65 g) was dissolved in ethanol (270 ml) at about 50°C, then left to stand for 2 days at room temperature. The precipitate was collected by filtration and dissolved in ethanol (l80 ml), then left to stand overnight at room temperature. The precipitate was collected by filtration and washed with ethanol, then dried to give (+)-di-p-toluoyl-D-tartaric acid salt of l-methyl-l-phenyl-l,2,3,4-tetrahydroisoquinoline (4.42 g).
   While, the filtrate was evaporated in vacuo and the residue was dissolved in ethyl acetate. The solution was adjusted to pH 8.0 with K₂CO₃ aqueous solution and organic layer was separated and washed with water and sodium chloride aqueous solution, dried over magnesium sulfate and evaporated in vacuo. The residue (6.0 g) and (-)-di-p-toluoyl-D-tartaric acid monohydrate (l0.86 g) was dissolved in ethanol (l70 ml), then left to stand overnight at room temperature. The precipitate was collected by filtration and washed with ethanol, then dried to give (-)-di-p-toluoyl-D-tartaric acid salt of l-methyl-l-phenyl-l,2,3,4-tetrahydroisoquinoline (10.0 g).
(2) (+)-Di-p-toluoyl-D-tartaric acid salt of l-methyl-l-phenyl-l,2,3,4-tetrahydroisoquinoline (3.0 g) was dissolved in ethyl acetate and the solution was adjusted to pH 8.0 with saturated K₂CO₃ aqueous solution. The organic layer was separated and washed with water and sodium chloride aqueous solution, dried over magnesium sulfate and evaporated in vacuo. The residue was dissolved in ethanol and added 7M ethanol solution of hydrogen chloride. The precipitate was collected by filtration, washed with ethanol and diethyl ether, then dried to give (+)-l-methyl-l-phenyl-l,2,3,4-tetrahydroisoquinoline hydrochloride (l.20 g).
   (-)-l-Methyl-l-phenyl-l,2,3,4-tetrahydroisoquinoline hydrochloride (1.70 g) was obtained according to a similar manner to that of (+) isomer from (-)-di-p-toluoyl-D-tartaric acid salt of l-methyl-l-phenyl-l,2,3,4-tetrahydroisoquinoline.

### Physicochemical Properties of Obtained Compounds

(+)-Di-p-toluoyl-D-tartaric acid salt of l-methyl-l-phenyl-l,2,3,4-tetrahydroisoquinoline
mp : l83-l84°C
[α]$\frac{\text{20}}{\text{D}}$ = -19.5° (C=l, EtOH) (optical rotation was measured after this tartaric acid salt was converted to free form)
(-)-Di-p-toluoyl-D-tartaric acid salt of l-methyl-l-phenyl-l,2,3,4-tetrahydroisoquinoline
mp : l84-l85°C
[α]$\frac{\text{20}}{\text{D}}$ = +17.9° (C=l, EtOH) (optical rotation was measured after this tartaric acid salt was converted to free form)
(+)-l-Methyl-l-phenyl-l,2,3,4-tetrahydroisoquinoline hydrochloride
mp : 302-303°C
[α]$\frac{\text{20}}{\text{D}}$ = +l0.9° (C=l, EtOH)
IR (Nujol) : l585, l500, l260 cm⁻¹
NMR (DMSO-d₆, δ) : 2.20 (3H, s), 2.90-3.70 (4H, m), 7.l5-7.50 (9H, m)
Mass : 222 (M⁺-l)
(-)-l-Methyl-l-phenyl-l,2,3,4-tetrahydroisoquinoline hydrochloride
mp : 300-30l°C
[α]$\frac{\text{20}}{\text{D}}$= -l3.30° (C=l, EtOH)
IR (Nujol) : l585, l500, l260 cm⁻¹
NMR (DMSO-d₆, δ) : 2.20 (3H, s), 2.90-3.70 (4H, m), 7.l5-7.50 (9H, m)
Mass : 222 (M⁺-l)

| Elemental Analysis for C₁₆H₁₇N^{.}HCl | | | |
|---|---|---|---|
| Calcd. | C 73.98, | H 6.98, | N 5.39 |
| Found | C 74.06, | H 6.95, | N 5.37 |

The following compounds (Preparations 6 to 20) were obtained according to a similar manner to that of Preparation l.

### Preparation 6

l-(p-Tolyl)-2-benzyl-3,4-dihydroisoquinolinium iodide
mp : 203-204°C
IR (Nujol) : l620, l6l0, l560 cm⁻¹
NMR (DMSO-d₆, δ) : 2.45 (3H, s), 3.28 (2H, d, J=7Hz), 4.l5 (2H, d, J=7Hz), 5.l0 (2H, s), 7.04-7.86 (l3H, m)

### Preparation 7

l-(4-Methoxyphenyl)-2-benzyl-3,4-dihydroisoquinolinium iodide
mp : 76-78°C
IR (Nujol) : l600, l560, l5l0 cm⁻¹
NMR (DMSO-d₆, δ) : 3.24 (2H, t, J=7Hz), 3.88 (3H, s), 4.l3 (2H, t, J=7Hz), 5.l4 (2H, s), 7.08-7.86 (l3H, m)

### Preparation 8

l-(3-Fluorophenyl)-2-benzyl-3,4-dihydroisoquinolinium iodide
mp : l78-l80°C
IR (Nujol) : l6l5, l600, l580, l560 cm⁻¹
NMR (DMSO-d₆, δ) : 3.32 (2H, t, J=7Hz), 4.l7 (2H, t, J=7Hz), 5.09 (2H, s), 7.07-7.88 (l3H, m)

### Preparation 9

l-(4-Fluorophenyl)-2-benzyl-3,4-dihydroisoquinolinium iodide
mp : l95-l96°C
NMR (DMSO-d₆, δ) : 3.40 (2H, t, J=9Hz), 4.28 (2H, t, J=9Hz), 5.2l (2H, s), 7.08-8.l4 (l3H, m)

### Preparation 10

l-(2-Chlorophenyl)-2-benzyl-3,4-dihydroisoquinolinium iodide
mp : l8l-l82°C
IR (Nujol) : l620, l600, l565 cm⁻¹
NMR (DMSO-d₆, δ) : 3.256-3.46 (2H, m), 4.l4-4.44 (2H, m), 5.04 (lH, d, J=l5Hz), 5.25 (lH, d, J=l5Hz), 7.04-8.l0 (l3H, m)

### Preparation 11

l-(4-Chlorophenyl)-2-benzyl-3,4-dihydroisoquinolinium iodide
mp : l88-l89°C
IR (Nujol) : l6l5, l600, l565, l480 cm⁻¹
NMR (DMSO-d₆, δ) : 3.32 (2H, t, J=7Hz), 4.l7 (2H, t, J=7Hz), 5.09 (2H, s), 7.06-7.88 (l3H, m)

### Preparation 12

l-Phenyl-2-benzyl-4-methyl-3,4-dihydroisoquinolinium iodide
mp : l95-l97°C
IR (Nujol) : l6l0, l600, l565, l480 cm⁻¹
NMR (DMSO-d₆, δ) : l.l3 and l.l6 (total 3H, each s), 3.35-3.59 (lH, m), 4.00 (lH, dd, J=7Hz and l4Hz), 4.25 (lH, dd, J=6Hz and l4Hz), 5.03 (lH, d, J=l5Hz), 5.l6 (lH, d, J=l5Hz), 7.03-7.92 (l4H, m)

### Preparation l3

l-Phenyl-2-benzyl-7-methyl-3,4-dihydroisoquinolinium iodide
mp : 2ll-2l2°C
IR (Nujol) : l620, l560 cm⁻¹
NMR (DMSO-d₆, δ) : 2.25 (3H, s), 3.26 (2H, t, J=7Hz), 4.l5 (2H, t, J=7Hz), 5.06 (2H, s), 6.82 (lH, s), 7.40-7.8l (l2H, m)

### Preparation 14

l-Phenyl-2-benzyl-3-acetoxymethyl-3,4-dihydroisoquinolinium iodide
mp : l76-l77°C
IR (Nujol) : l740, l600, l560 cm⁻¹
NMR (DMSO-d₆, δ) : l.79 (3H, s), 3.35 (lH, s), 3.59-3.7l (lH, m), 4.37-4.58 (3H, m), 5.25 (2H, s), 6.99-7.9l (l4H, m)

### Preparation l5

l-Phenyl-2-benzyl-5-chloro-3,4-dihydroisoquinolinium iodide
mp : 244-246°C
IR (Nujol) : l620, l585, l560, l495 cm⁻¹
NMR (DMSO-d₆, δ) : 3.53 (2H, t, J=8Hz), 4.22 (2H, t, J=8Hz), 5.ll (2H, s), 7.0l-8.02 (l3H, m)

### Preparation 16

l-Phenyl-2-benzyl-7-chloro-3,4-dihydroisoquinolinium iodide
mp : 236-237°C
NMR (DMSO-d₆, δ) : 3.37 (2H, t, J=8Hz), 4.29 (2H, t, J=8Hz), 5.l6 (2H, s), 6.98-8.08 (l3H, m)

### Preparation 17

l-Phenyl-2-benzyl-6-ethoxycarbonylamino-3,4-dihydroisoquinolinium bromide
mp : 2l8-2l9°C (dec.)
IR (Nujol) : 3400, 3200, 3l40, l740, l600 cm⁻¹
NMR (DMSO-d₆, δ) : l.27 (3H, t, J=7Hz), 3.23 (2H, t, J=7Hz), 4.09 (2H, t, J=7Hz), 4.l8 (2H; t, J=7Hz), 4.98 (2H, s), 6.95-7.7l (l3H, m)

### Preparation 18

l-Cyclohexyl-2-benzyl-3,4-dihydroisoquinolinium iodide
mp : l62-l64°C
IR (Nujol) : l600, l560, l495 cm⁻¹
NMR (DMSO-d₆, δ) : l.09-2.08 (l0H, m), 3.l0 (2H, t, J=7Hz), 3.35-3.44 (lH, m), 3.85 and 4.00 (total 2H, each t, J=7Hz), 5.50 (2H, s), 7.48-8.37 (9H, m)

### Preparation 19

l-(2-Thienyl)-2-(4-methoxybenzyl)-3,4-dihydroisoquinolinium iodide
IR (Film) : l6l0, l255 cm⁻¹
NMR (DMSO-d₆, δ) : 3.20 (2H, t, J=7Hz), 3.75 (3H, s), 4.08 (2H, t, J=7Hz), 5.l7 (2H, s), 6.87-8.23 (llH, m)

### Preparation 20

l-Phenyl-2-benzyl-4,5-dihydro-3H-2-benzazepinium bromide
mp : 236-238°C
IR (Nujol) : l6l0, l595, l565 cm⁻¹
NMR (DMSO-d₆, δ) : l.96 (2H, t, J=7Hz), 2.90 (2H, t, J=7Hz), 3.89 (2H, t, J=7Hz), 5.35 (2H, s), 6.99-7.84 (l4H, m)
The following compounds (Preparations 2l to 36) were obtained according to a similar manner to that of Preparation 3.

### Preparation 21

l-Methyl-l-(p-tolyl)-2-benzyl-l,2,3,4-tetrahydroisoquinoline
mp : l39-l40°C
IR (Nujol) : l600, l5l0, l490 cm⁻¹
NMR (CDCl₃, δ) : l.78 (3H, s), 2.60 (3H, s), 2.60-3.l6 (4H, m), 3.27 (lH, d, J=l4Hz), 3.57 (lH, d, J=l4Hz), 6.67-7.49 (l3H, m)

### Preparation 22

l-Methyl-l-(4-methoxyphenyl)-2-benzyl-l,2,3,4-tetrahydroisoquinoline
mp : ll3-ll4°C
IR (Nujol) : l600, l505, l490 cm⁻¹
NMR (CDCl₃, δ) : l.76 (3H, s), 2.63-3.l5 (4H, m), 3.25 (lH, d, J=l4Hz), 3.59 (lH, d, J=l4Hz), 3.73 (3H, s), 6.67-7.52 (13H, m)

### Preparation 23

l-Methyl-l-(3-fluorophenyl)-2-benzyl-l,2,3,4-tetrahydroisoquinoline
mp : 83-85°C
IR (Nujol) : l605, l590, l485 cm⁻¹

### Preparation 24

l-Methyl-l-(4-fluorophenyl)-2-benzyl-l,2,3,4-tetrahydroisoquinoline
mp : l54-l55°C
NMR (CDCl₃, δ) : l.86 (3H, s), 2.65-3.06 (4H, m), 3.33 (lH, d, J=l4Hz), 3.70 (lH, d, J=l4Hz), 6.68-7.78 (l3H, m)
Mass (M/Z) : 33l (M⁺)

### Preparation 25

l-Methyl-l-(2-chlorophenyl)-2-benzyl-l,2,3,4-tetrahydroisoquinoline hydrochloride
NMR (DMSO-d₆, δ) : 2.l0 (3H, s), 3.00-4.20 (6H, m), 7.l5-8.00 (l3H, m)
Mass (M/Z) : 347 (M⁺)

### Preparation 26

l-Methyl-l-(4-chlorophenyl)-2-benzyl-l,2,3,4-tetrahydroisoquinoline
mp : l34-l35°C
NMR (CDCl₃, δ) : l.82 (3H, s), 2.60-3.05 (4H, m), 3.28 (lH, d, J=l4Hz), 3.65 (lH, d, J=l4Hz), 6.58-7.68 (13H, m)
Mass (M/Z) : 347 (M⁺)

### Preparation 27

l-Methyl-l-phenyl-2-benzyl-4-methyl-l,2,3,4-tetrahydroisoquinoline
mp : l25-l26°C
IR (Nujol) : l600, l580, l490 cm⁻¹
NMR (CDCl₃, δ) : l.2l and l.24 (total 3H, each s), l.76 (3H, s), 2.50-3.05 (3H, m), 3.l9 (lH, d, J=l3Hz), 3.63 (lH, d, J=l3Hz), 6.63-7.67 (l4H, m)

### Preparation 28

l-Methyl-l-phenyl-2-benzyl-7-methyl-l,2,3,4-tetrahydroisoquinoline
mp : 94-95°C
IR (Nujol) : l600, l490, l420 cm⁻¹
NMR (DMSO-d₆, δ) : l.79 (3H, s), 2.l2 (3H, s), 2.44-3.l2 (4H, m), 3.l7 (2H, d, J=l4Hz), 3.54 (2H, d, J=l4Hz), 6.49 (lH, s), 6.83-7.62 (l2H, m)

### Preparation 29

l-Methyl-l-phenyl-2-benzyl-3-hydroxymethyl-l,2,3,4-tetrahydroisoquinoline
IR (Film) : 3550, 3400, l600, l495, l450 cm⁻¹
NMR (CDCl₃, δ) : l.76 and l.82 (total 3H, s), 2.70-4.02 (6H, m), 6.62-7.52 (14H, m)
Mass (M/Z) : 342 (M⁺-l)

### Preparation 30

l-Methyl-l-phenyl-2-benzyl-5-chloro-l,2,3,4-tetrahydroisoquinoline
mp : 98-l00°C
IR (Nujol) : l565, l490 cm⁻¹
NMR (CDCl₃, δ) : l.80 (3H, s), 2.73-2.95 (4H, m), 3.26 (lH, d, J=l4Hz), 3.55 (lH, d, J=l4Hz), 5.58-7.59 (l3H, m)

### Preparation 3l

l-Methyl-l-phenyl-2-benzyl-7-chloro-l,2,3,4-tetrahydroisoquinoline
mp : 96-97°C
NMR (CDCl₃, δ) : l.85 (3H, s), 2.60-3.l5 (4H, m), 3.30 (lH, d, J=l4Hz), 3.62 (lH, d, J=l4Hz), 6.70-7.72 (l3H, m)
Mass (M/Z) : 347 (M⁺)

### Preparation 32

l-Methyl-l-phenyl-2-benzyl-6-ethoxycarbonylamino-l,2,3,4-tetrahydroisoquinoline
IR (Film) : 3420, l720 cm⁻¹
NMR (CDCl₃, δ) : l2.5 (3H, t, J=7Hz), l.75 (3H, s), 2.55-3.06 (4H, m), 3.24 (lH, d, J=l4Hz), 3.53 (lH, d, J=l4Hz), 4.l8 (2H, d, J=7Hz), 6.52-7.62 (l4H, m)
Mass (M/Z) : 385 (M⁺ - 15)

### Preparation 33

l-Methyl-l-cyclohexyl-2-benzyl-l,2,3,4-tetrahydroisoquinoline
IR (Film) : 2940, l600, l490, l450 cm⁻¹
NMR (CDCl₃, δ) : l.l5-2.l0 (l4H, m), 2.56-3.08 (4H, m), 3.40 (lH, d, J=l5Hz), 4.22 (lH, d, J=l5Hz), 7.l2-7.54 (9H, m)
Mass (M/Z) : 302 (M⁺ + l)

### Preparation 34

l-Methyl-l-(2-furyl)-2-benzyl-l,2,3,4-tetrahydroisoquinoline
NMR(CDCl₃, δ) : l.88 (3H, s), 2.94 (4H, s), 3.48 (lH, d, J=l4Hz), 3.75 (lH, d, J=l4Hz), 6.44 (2H, s), 7.05-7.54 (l0H, m)
Mass (M/Z) : 303 (M⁺)

### Preparation 35

l-Methyl-l-(2-thienyl)-2-(4-methoxybenzyl)-l,2,3,4-tetrahydroisoquinoline
mp : 80-82°C
IR (Nujol) : l6l0, l240, l035, 700 cm⁻¹
NMR (CDCl₃, δ) : l.8l (3H, s), 2.5-3.2 (4H, m), 3.30 (lH, d, J=l4Hz), 3.68 (lH, d, J=l4Hz), 3.72 (3H, s), 6.7-7.4 (llH, m)

### Preparation 36

l-Methyl-l-phenyl-2-benzyl-2,3,4,5-tetrahydro-lH-2-benzazepine
mp : l34-l35°C
IR (Nujol) : l600, l485 cm⁻¹
NMR (CDCl₃, δ) : l.ll-l.29 (lH, m), l.73-l.79 (lH, m), l.89 (3H, s), 2.56-2.84 (3H, m), 3.35-3.46 (lH, m), 3.35 (lH, d, J=7Hz), 3.80 (lH, d, J=7Hz), 7.l5-7.44 (l4H, m)
Mass (M/Z) : 3l2 (M⁺ - 15)

### Preparation 37

l-Methyl-l-(3-thienyl)-2-(4-methoxybenzyl)-l,2,3,4-tetrahydroisoquinoline was prepared by reacting l-(3-thienyl)-3,4-dihydroisoquinoline with 4-methoxybenzyl bromide according to a similar manner to that of Preparation l, and then by reacting the obtained l-(3-thienyl)-2-(4-methoxybenzyl)-3,4-dihydroisoquinolinium bromide with 3 M diethyl ether solution of methylmagnesium bromide according to a similar manner to that of Preparation 3.
IR (Film) : l6l0, l040 cm⁻¹
NMR (CDCl₃, δ) : l.78 (3H, s), 2.8-3.l (4H, m), 3.20 (lH, d, J=l3.5Hz), 3.28 (lH, d, J=l3.5Hz), 3.78 (3H, s), 6.9-7.2 (llH, m)

### Preparation 38

The mixture of l-phenyl-3,4-dihydroisoquinoline (292.0 g) and benzyl bromide (l76 ml) in acetonitrile (3 ℓ) was refluxed for l hour. After cooling the mixture to room temperature, the solvent was evaporated in vacuo. The residue was triturated with diethyl ether (l ℓ) to give l-phenyl-2-benzyl-3,4-dihydroisoquinolinium bromide (47l.2 g).
mp : l8l-l83°C
IR (Nujol) : l620, l600 cm⁻¹
NMR (DMSO-d₆, δ) : 3.24 (2H, t, J=5Hz), 4.l4 (2H, t, J=5Hz), 5.02 (2H, s), 6.88-7.86 (l4H, m)

### Preparation 39

To a suspension of l-phenyl-2-benzyl-3,4-dihydroisoquinolinium bromide (353.l g) in tetrahydrofuran (3.5 ℓ) was added 2.0 M tetrahydrofuran solution of methylmagnesium bromide (700 ml) for l.5 hours with stirring at room temperature. After additional stirring for l.5 hours, the mixture was poured into a solution of ammonium chloride (300 g) in water (650 ml) under ice cooling. After separation, the aqueous layer was extracted with ethyl acetate (3.5 ℓ). The combined organic layers were washed with water (l ℓ), sodium chloride aqueous solution (l ℓ) and dried over magnesium sulfate (l25 g) and evaporated in vacuo. The residue was crystallized from ethanol (500 ml) to give l-methyl-l-phenyl-2-benzyl-l,2,3,4-tetrahydroisoquinoline (254.0 g).
mp : l02-l03°C
IR (Nujol) : l600, l580, l485 cm⁻¹
NMR (CDCl₃, δ) : l.78 (3H, s), 2.58-3.l0 (4H, m), 3.22 (lH, d, J=l4Hz), 3.56 (lH, d, J=l4Hz), 6.60-7.65 (l4H, m)
Mass : 3l3 (M⁺)
The following compounds (Preparations 40 to 42) were obtained according to a similar manner to that of Preparation l.

### Preparation 40

l-Phenyl-2-ethyl-3,4-dihydroisoquinolinium iodide
mp : l65-l66°C (dec.)
IR (Nujol) : l620, l600, l560 cm⁻¹
NMR (DMSO-d₆, δ) : l.35 (3H, t, J=7Hz), 3.35-3.45 (2H, m), 3.78 (2H, t, J=7Hz)

### Preparation 4l

l-(2-Thienyl)-2-methyl-3,4-dihydroisoquinolinium iodide
mp : l80-l8l°C (dec.)
IR (Nujol) : l590, 720 cm⁻¹
NMR (DMSO-d₆, δ) : 3.25-3.6 (2H, m), 3.75 (3H, s), 4.2-4.55 (2H, m), 7.2-8.0 (7H, m)

### Preparation 42

l-(2-Thienyl)-2-ethyl-3,4-dihydroisoquinolinium iodide
mp : l94-l95°C
IR (Nujol) : l600, 800, 740 cm⁻¹
NMR (DMSO-d₆, δ) : l.43 (3H, t, J=8Hz), 3.2-3.5 (2H, m), 3.7-4.2 (2H, m), 4.25 (2H, t, J=8Hz), 7.0-8.3 (7H, m)
The following compounds (Examples 5 to l8) were obtained according to a similar manner to that of Example 1.

### Example 5

l-Methyl-l-(p-tolyl)-l,2,3,4-tetrahydroisoquinoline hydrochloride
mp : 294-295°C
IR (Nujol) : l575, l5l0, l490 cm⁻¹
NMR (DMSO-d₆, δ) : 2.l2 (3H, s), 2.32 (3H, s), 2.80-3.85 (4H, m), 7.l2-7.40 (8H, m), l0.00 (lH, s), l0.55 (lH, s)
Mass (M/Z) : 236 (M⁺ - l)

### Example 6

l-Methyl-l-(4-methoxyphenyl)-l,2,3,4-tetrahydroisoquinoline hydrochloride
mp : 25l-253°C
IR (Nujol) : l6l0, l585, l535 cm⁻¹
NMR (DMSO-d₆, δ) : 2.l5 (3H, s), 2.85-3.50 (4H, m), 3.80 (3H, s), 6.90-7.50 (8H, m)
Mass (M/Z) : 252 (M⁺ - l)

| Elemental analysis for C₁₇H₁₉NO^{.}HCl | | | |
|---|---|---|---|
| Calcd. | C 70.46, | H 6.96, | N 4.83 |
| Found | C 70.06, | H 7.00, | N 4.80 |

### Example 7

l-Methyl-l-(3-fluorophenyl)-l,2,3,4-tetrahydroisoquinoline hydrochloride
mp : 285-287°C
IR (Nujol) : l605, l585, l490 cm⁻¹
NMR (DMSO-d₆, δ) : 2.06 (3H, s), 2.80-3.40 (4H, m), 6.95-7.48 (8H, m), 10.00 (lH, broad), 10.42 (lH, broad)
Mass (M/Z) : 240 (M⁺ - l)

| Elemental Analysis for C₁₆H₁₆FN^{.}HCl | | | |
|---|---|---|---|
| Calcd. | C 69.l9, | H 6.l7, | N 5.04 |
| Found | C 68.79, | H 6.45, | N 4.97 |

### Example 8

l-Methyl-l-(4-fluorophenyl)-l,2,3,4-tetrahydroisoquinoline hydrochloride
mp : 254-256°C
IR (Nujol) : l600, l585, l5l0, l490 cm⁻¹
NMR (DMSO-d₆, δ) : 2.l8 (3H, s), 2.70-3.55 (4H, m), 7.l0-7.60 (8H, m), 9.80-l0.80 (2H, broad)
Mass (M/Z) : 240 (M⁺ - l)

| Elemental Analysis for C₁₆H₁₆FN^{.}HCl | | | |
|---|---|---|---|
| Calcd. | C 69.l9, | H 6.l7, | N 5.04 |
| Found | C 68.84, | H 6.09, | N 4.96 |

### Example 9

l-Methyl-l-(2-chlorophenyl)-l,2,3,4-tetrahydroisoquinoline hydrochloride
mp : 264-265°C (dec.)
IR (Nujol) : l580, l490, l435 cm⁻¹
NMR (DMSO-d₆, δ) : 2.26 (3H, s), 3.00-3.70 (4H, m), 6.58-8.l0 (8H, m)
Mass (M/Z) : 257 (M⁺)

| Elemental Analysis for C₁₆H₁₆ClN^{.}HCl^{.}l/2H₂O | | | |
|---|---|---|---|
| Calcd. | C 63.38, | H 5.98, | N 4.62 |
| Found | C 63.78, | H 6.l4, | N 4.37 |

### Example 10

l-Methyl-l-(4-chlorophenyl)-l,2,3,4-tetrahydroisoquinoline hydrochloride
mp : 286-287°C (dec.)
IR (Nujol) : l580, l490, l420, l400 cm⁻¹
NMR (DMSO-d₆, δ) : 2.l2 (3H, s), 2.80-3.50 (4H, m), 7.00-7.53 (8H, m), l0.l2 (lH, broad), l0.55 (lH, broad)
Mass (M/Z) : 256 (M⁺ - l)

### Example 11

l-Methyl-l-phenyl-4-methyl-l,2,3,4-tetrahydroisoquinoline hydrochloride
mp : 245°C (dec.)
IR (Nujol) : l585, l490 cm⁻¹
NMR (DMSO-d₆, δ) : l.25 (3H, d, J=7Hz), 2.22 (3H, s), 3.20-3.78 (3H, m), 7.26-7.62 (9H, m), l0.00-l0.70 (2H, broad)
Mass (M/Z) : 236 (M⁺ - l)

| Elemental Analysis for C₁₇H₁₉N^{.}HCl | | | |
|---|---|---|---|
| Calcd. | C 74.58, | H 7.36, | N 5.l2 |
| Found | C 74.37, | H 7.l2, | N 5.l5 |

### Example 12

l-Methyl-l-phenyl-7-methyl-l,2,3,4-tetrahydroisoquinoline hydrochloride
mp : 304-305°C (dec.)
IR (Nujol) : l590, l500 cm⁻¹
NMR (DMSO-d₆, δ) : 2.20 (3H, s), 2.32 (3H, s), 3.05-3.35 (4H, m), 7.02 (lH, s), 7.24 (2H, s), 7.45 (5H, s), l0.04 (lH, s), l0.55 (lH, s)
Mass (M/Z) : 236 (M⁺ - l)

| Elemental Analysis for C₁₇H₁₉N^{.}HCl^{.}l/4H₂O | | | |
|---|---|---|---|
| Calcd. | C 73.37, | H 7.42, | N 5.03 |
| Found | C 73.53, | H 7.47, | N 5.00 |

### Example l3

l-Methyl-l-phenyl-3-hydroxymethyl-l,2,3,4-tetrahydroisoquinoline
mp : l25-l27°C
IR (Nujol) : 3250, l595, l585, l490 cm⁻¹
NMR (CDCl₃, δ) : l.85 (3H, s), 2.58-3.00 (3H, m), 3.30-3.98 (2H, m), 7.l2-7.22 (9H, m)
Mass (M/Z) : 252 (M⁺ - l)

| Elemental Analysis for C₁₇H₁₉NO | | | |
|---|---|---|---|
| Calcd. | C 80.57, | H 7.56, | N 5.53 |
| Found | C 80.2l, | H 7.35, | N 5.4l |

### Example l4

l-Methyl-l-phenyl-5-chloro-l,2,3,4-tetrahydroisoquinoline hydrochloride
mp : 3l5°C (dec.)
IR (Nujol) : l575, l490 cm⁻¹
NMR (DMSO-d₆, δ) : 2.l6 (3H, s), 2.90-3.40 (4H, m), 7.05-7.56 (8H, m)

| Elemental Analysis for C₁₆H₁₆ClN^{.}HCl | | | |
|---|---|---|---|
| Calcd. | C 65.32, | H 5.82, | N 4.76 |
| Found | C 65.l8, | H 5.80, | N 4.56 |

### Example l5

l-Methyl-l-phenyl-7-chloro-l,2,3,4-tetrahydroisoquinoline hydrochloride
mp : 3l9-320°C (dec.)
IR (Nujol) : l585, l480 cm⁻¹
NMR (DMSO-d₆, δ) : 2.24 (3H, s), 3.l0-3.40 (4H, m), 7.38-7.62 (8H, m)

| Elemental Analysis for C₁₆H₁₆ClN^{.}HCl^{.}l/3H₂O | | | |
|---|---|---|---|
| Calcd. | C 64.0l, | H 5.93, | N 4.67 |
| Found | C 64.05, | H 5.99, | N 4.50 |

### Example l6

l-Methyl-l-phenyl-6-ethoxycarbonylamino-l,2,3,4-tetrahydroisoquinoline hydrochloride
mp : 260-26l°C (dec.)
IR (Nujol) : 3260, l730, l600, l585 cm⁻¹
NMR (DMSO-d₆, δ) : l.35 (3H, t, J=7Hz), 2.28 (3H, s), 2.95-3.40 (4H, m), 4.25 (2H, q, J=7Hz), 7.l0-7.65 (8H, m), 9.82 (lH, s)
Mass (M/Z) : 3ll (M⁺ + l)

### Example 17

l-Methyl-l-cyclohexyl-l,2,3,4-tetrahydroisoquinoline hydrochloride
mp : 280-28l°C
IR (Nujol) : l590, l495, l4l5 cm⁻¹
NMR (DMSO-d₆, δ) : l.00-2.35 (l4H, m), 2.80-3.55 (4H, m), 7.22-7.40 (4H, m)
Mass (M/Z) : 228 (M⁺ - l)

### Example l8

l-Methyl-l-(2-furyl)-l,2,3,4-tetrahydroisoquinoline hydrochloride
mp : 223-225°C
IR (Nujol) : l580, l485, l425 cm⁻¹
NMR (DMSO-d₆, δ) : 2.l2 (3H, s), 3.l6-3.58 (4H, m), 6.40-6.58 (2H, m), 7.l8-7.36 (4H, m), 7.75 (lH, broad), l0.08 (lH, broad), l0.82 (lH, broad)
Mass (M/Z) : 2l2 (M⁺ - l)

| Elemental Analysis for C₁₄H₁₅NO^{.}HCl | | | |
|---|---|---|---|
| Calcd. | C 67.33, | H 6.46, | N 5.6l |
| Found | C 66.60, | H 6.27, | N 5.45 |

### Example 19

To a solution of ceric ammonium nitrate (7 g) in a mixture off acetonitrile (30 ml) and water (l5 ml) was added l-methyl-l-(2-thienyl)-2-(4-methoxybenzyl)-l,2,3,4-tetrahydroisoquinoline (l.5 g). After stirring for l.5 hours at room temperature, the mixture was poured into a mixture of n-hexane (50 ml) and water (50 ml) with stirring. The separated aqueous layer was adjusted to pH l0 with saturated potassium carbonate aqueous solution and extracted with ethyl acetate (50 ml x 2). The organic layer was washed with sodium chloride aqueous solution, dried over magnesium sulfate and extracted in vacuo. The residue was recrystallized from 6N-hydrogen chloride ethanol solution (l ml) and diethyl ether (l0 ml) to give l-methyl-l-(2-thienyl)-l,2,3,4-tetrahydroisoquinoline hydrochloride (0.5l g).
mp : 264-265°C (dec.)
IR (Nujol) : l580, l260 cm⁻¹
NMR (DMSO-d₆, δ) : 2.l8 (3H, s), 2.8-3.5 (4H, m), 6.9-7.6 (7H, m), 9.8-l0.7 (2H, broad)

| Elemental Analysis for C₁₄H₁₅NS^{.}HCl | | | |
|---|---|---|---|
| Calcd. | C 63.26, | H 6.07, | N 5.27 |
| Found | C 62.68, | H 5.96, | N 5.07 |

### Example 20

l-Methyl-l-(3-thienyl)-l,2,3,4-tetrahydroisoquinoline hydrochloride was obtained according to a similar manner to that of Example 19.
mp : 280-28l°C (dec.)
IR (Nujol) : l580, ll65, 800 cm⁻¹
NMR (DMSO-d₆, δ) : 2.l2 (3H, s), 2.8-3.3 (4H, m), 7.l-7.3 (6H, m), 7.5-7.7 (lH, m), l0.05 (lH, broad), l0.33 (lH, broad)

| Elemental Analysis for C₁₄H₁₅NS^{.}HCl | | | |
|---|---|---|---|
| Calcd. | C 63.26, | H 6.06, | N 5.26 |
| Found | C 63.06, | H 5.64, | N 5.08 |

### Example 21

l-Methyl-l-(2-thienyl)-l,2,3,4-tetrahydroisoquinoline hydrochloride was subjected to optical resolution according to a similar manner to that of Example 4 to give the following pair of enantiomers.
i) (+)-l-Methyl-l-(2-thienyl)-l,2,3,4-tetrahydroisoquinoline hydrochloride
mp : 292-293°C
[α]$\frac{\text{25}}{\text{D}}$ =+ 8.9° (c=l, MeOH)
IR (Nujol) : 2700, l580, l260 cm⁻¹
NMR (DMSO-d₆, δ) : 2.22 (3H, s), 2.8-3.4 (4H, m), 7.0-7.7 (7H, m), 9.8-l0.9 (2H, broad)
ii) (-)-l-Methyl-l-(2-thienyl)-l,2,3,4-tetrahydroisoquinoline hydrochloride
mp : 289-290°C (dec.)
[α]$\frac{\text{25}}{\text{D}}$ = -l0.00° (c=l, EtOH)
IR (Nujol) : 2700, l580, l260 cm⁻¹
NMR (DMSO-d₆, δ) : 2.27 (3H, s), 3.0-3.4 (4H, m), 7.0-7.7 (7H, m), 9.8-l0.9 (2H, broad)

| Elemental Analysis for C₁₄H₁₆ClNS^{.}l/4H₂O | | | |
|---|---|---|---|
| Calcd. | C 62.2l, | H 6.l5, | N 5.l8 |
| Found | C 62.58, | H 6.26, | N 5.l3 |

### Example 22

To a suspension of l-phenyl-2-methyl-3,4-dihydroisoquinolinium iodide (l.0 g) in tetrahydrofuran (15 ml) was added 3M diethyl ether solution of methyl magnesium bromide (l.9 ml) with stirring at room temperature. After stirring for 30 minutes, the mixture was poured into saturated ammonium chloride aqueous solution and ethyl acetate. The organic layer was washed with water, sodium chloride aqueous solution and dried over magnesium sulfate, and evaporated in vacuo. The residue was recrystallized from n-hexane to give l,2-dimethyl-l-phenyl-l,2,3,4-tetrahydroisoquinoline (0.6 g).
mp : 39-40°C
IR (Nujol) : l600, l590, l490 cm⁻¹
NMR (CDCl₃, δ) : l.70 (3H, s), 2.l5 (3H, s), 2.68-3.25 (4H, m), 6.62-7.50 (9H, m)
Mass (M/Z) : 237 (M⁺)
The following compounds (Examples 23 to 25) were obtained according to a similar manner to that of Example 22.

### Example 23

l-Methyl-l-phenyl-2-ethyl-l,2,3,4-tetrahydroisoquinoline hydrochloride
mp : l93-l94°C
IR (Nujol) : l490, l420, l400 cm⁻¹
NMR (DMSO-d₆, δ) : l.24-l.34 (3H, m), 2.02-2.35 (total 3H, s), 2.90-3.70 (6H, m), 6.62-7.64 (9H, m), l0.50-l0.80 (lH, broad), ll.30-ll.60 (lH, broad)
Mass (M/Z) : 25l (M⁺)

| Elemental Analysis for C₁₈H₂₁N^{.}HCl^{.}l/4H₂O | | | |
|---|---|---|---|
| Calcd. | C 73.95, | H 7.75, | N 4.79 |
| Found | C 74.2l, | H 7.44, | N 4.80 |

### Example 24

l,2-Dimethyl-l-(2-thienyl)-l,2,3,4-tetrahydroisoquinoline hydrochloride
mp : 220-222°C
IR (Nujol) : 2320, l260, 725 cm⁻¹
NMR (D₂O, δ) : 2.27 (3H, s), 2.89 (3H, s), 3.2-3.8 (4H, m), 7.0-7.7 (7H, m)

| Elemental Analysis for C₁₅H₁₈ClNS | | | |
|---|---|---|---|
| Calcd. | C 64.38, | H 6.48, | N 5.0l |
| Found | C 63.83, | H 6.44, | N 4.94 |

### Example 25

l-Methyl-l-(2-thienyl)-2-ethyl-l,2,3,4-tetrahydroisoquinoline hydrochloride
mp : l98-200°C
IR (Nujol) : ll75, 740 cm⁻¹
NMR (DMSO-d₆, δ) : l.34 (3H, t, J=7.5Hz), 2.22 (3H, s), 3.0-3.9 (6H, m), 6.9-7.7 (7H, m)

### Example 26

A solution of l-phenyl-3,4-dihydroisoquinoline (l.0 g) and allyl bromide (0.44 ml) in acetonitrile (l0 ml) was refluxed for 2 hours. After cooling it to room temperature, the solvent was evaporated in vacuo and then the residue was stirred in diethyl ether (l5 ml) at room temperature. To the mixture was added 3 M diethyl ether solution of methylmagnesium bromide (2.0 ml). After l hour, the mixture was poured into aqueous ammonium chloride solution. The organic layer was washed with water, sodium chloride aqueous solution and dried over magnesium sulfate, and evaporated in vacuo to give l-methyl-l-phenyl-2-allyl-l,2,3,4-tetrahydroisoquinoline (0.7 g).
IR (Film) : l640, l600, l490, l450 cm⁻¹
NMR (CDCl₃, δ) : l.69 (3H, s), 2.70-3.l5 (6H, m), 4.98-5.l9 (2H, m), 5.54-5.78 (lH, m), 6.64-7.54 (9H, m)
Mass (M/Z) : 263 (M⁺)

### Example 27

l-Methyl-l-phenyl-2-phenethyl-l,2,3,4-tetrahydroisoquinoline hydrochloride was prepared by reacting l-phenyl-3,4-dihydroisoquinoline with phenethyl bromide, then 3 M diethyl ether solution of methylmagnesium bromide according to a similar manner to that of Example 26, and then converting the resultant compound to its hydrochloride according to a conventional manner.
mp : 255-256°C
IR (Nujol) : l600, l580, l490, l420 cm⁻¹
NMR (DMSO-d₆, δ) : 2.l5 and 2.48 (total 3H, s), 3.05-3.50 (4H, m), 3.55-4.20 (4H, m), 7.l0-8.00 (l4H, m)
Mass (M/Z) : 325 (M⁺ - 15)

| Elemental Analysis for C₂₄H₂₅N^{.}HCl^{.}0.lH₂O | | | |
|---|---|---|---|
| Calcd. | C 78.8l, | H 7.22, | N 3.82 |
| Found | C 78.77, | H 7.30, | N 3.79 |

### Example 28

To a solution of (+)-l-methyl-l-phenyl-l,2,3,4-tetrahydroisoquinoline (l.0 g) in N,N-dimethylformamide (l0 ml) was added sodium hydride (0.l9 g) in ice-bath with stirring. After stirring for 30 minutes at room temperature, to the mixture was added methyl iodide (0.3l ml). And after stirring for an hour at room temperature, the mixture was poured into ice-water and extracted with ethyl acetate. The organic layer was washed with water, sodium chloride aqueous solution, dried over magnesium sulfate and evaporated in vacuo to give (+)-l,2-dimethyl-l-phenyl-l,2,3,4-tetrahydroisoquinoline (0.6 g).
[α]$\frac{\text{20}}{\text{D}}$ = +105.6° (c=l.3 EtOH)
IR (Film) : l600, l585, l495, l450 cm⁻¹
NMR (CDCl₃, δ) : l.64 (3H, s), 2.ll (3H, s), 2.72-3.30 (4H, m), 6.62-7.44 (9H, m)
Mass (M/Z) : 237 (M⁺)

### Example 29

To a solution of l-methyl-l-phenyl-l,2,3,4-tetrahydroisoquinoline (l.0 g) in methylene chloride (l0 ml) was added chloroacetic anhydride (2.9 g) and the mixture was stirred overnight at room temperature. The solvent was evaporated in vacuo. The residue was poured into saturated aqueous solution of sodium bicarbonate and extracted with ethyl acetate. The organic layer was washed with water, sodium chloride aqueous solution and dried over magnesium sulfate, evaporated in vacuo. The resultant precipitate was recrystallized from diethyl ether and dried in vacuo to give l-methyl-l-phenyl-2-(2-chloroacetyl)-l,2,3,4-tetrahydroisoquinoline (0.77 g).
mp : l50-l52°C
IR (Nujol) : l665, l640 cm⁻¹
NMR (CDCl₃, δ) : 2.03 (3H, s), 3.04 (lH, d, J=llHz), 3.06 (lH, d, J=llHz), 3.75-4.l2 (4H, m), 6.62-7.34 (9H, m)
Mass (M/Z) : 299 (M⁺)

### Example 30

To a solution of l-methyl-l-phenyl-2-(2-chloroacetyl)-l,2,3,4-tetrahydroisoquinoline (l.0 g) in methylene chloride (l0 ml) was added piperidine (0.82 ml) under ice-cooling, and the mixture was stirred for 3 hours at room temperature. The reaction mixture was poured into water and extracted with methylene chloride. The organic layer was washed with saturated aqueous solution of sodium bicarbonate, sodium chloride aqueous solution and dried over magnesium sulfate, evaporated in vacuo. The residue was dissolved in ethanol (5 ml) and to the solution was added 6 M ethanol solution of hydrogen chloride (l ml). The mixture was evaporated in vacuo and the residue was recrystallized from isopropyl alcohol and diisopropyl ether. The crystal was washed with diethyl ether and dried in vacuo to give l-methyl-l-phenyl-2-(2-piperidinoacetyl)-l,2,3,4-tetrahydroisoquinoline hydrochloride (0.4 g).
mp : l46-l47°C (dec.)
IR (Nujol) : 3340, l660 cm⁻¹
NMR (DMSO-d₆, δ) : l.60-l.72 (6H, m), 2.05 (3H, s), 2.95-3.34 (4H, m), 3.64-3.84 (2H, m), 4.34-4.57 (2H, m), 6.73-7.38 (9H, m), 9.30 (lH, s)
Mass (M/Z) : 348 (M⁺)

| Elemental Analysis for C₂₃H₂₈N₂O (free) | | | |
|---|---|---|---|
| Calcd. | C 79.27, | H 8.09, | N 8.03 |
| Found | C 79.20, | H 7.37, | N 8.0l |

### Example 31

A solution of l-methyl-l-phenyl-2-(2-piperidinoacetyl)-l,2,3,4-tetrahydroisoquinoline (l.0 g) in tetrahydrofuran (5 ml) was dropped into a suspension of lithium aluminum hydride (0.l6 g) in tetrahydrofuran (l5 ml) over a period of 30 minutes under refluxing. The reaction mixture was heated under refluxing for an additional l hour. After cooling it in an ice-bath, to the reaction mixture was added ethyl acetate (5 ml), water (l ml), 4N aqueous solution of sodium hydroxide (l ml) and magnesium sulfate (2 g), then the mixture was filtrated and evaporated in vacuo. The residue was dissolved in ethanol (l0 ml) and to the solution was added 6 M ethanol solution of hydrogen chloride (2 ml). The precipitate was collected by filtration and washed with diethyl ether, and dried in vacuo to give l-methyl-l-phenyl-2-(2-piperidinoethyl)-l,2,3,4-tetrahydroisoquinoline dihydrochloride (0.4 g).
mp : l30°C (dec.)
IR (Nujol) : 3380, l590 cm⁻¹
NMR (DMSO-d₆ + D₂O, δ) : l.40-l.70 (6H, broad), 2.l3 (3H, s), 2.68-2.86 (4H, broad), 3.22-3.63 (8H, broad), 6.70-7.56 (9H, m)
Mass (M/Z) : 334 (M⁺)

| Elemental Analysis for C₂₃H₃₀N₂^{.}2HCl | | | | |
|---|---|---|---|---|
| Calcd. | C 67.80, | H 7.9l, | N 6.87, | Cl l7.40 |
| Found | C 67.44, | H 7.7l, | N 6.46, | Cl l7.32 |

### Example 32

l-Methyl-l-phenyl-2,3,4,5-tetrahydro-lH-2-benzazepine hydrochloride was obtained according to a similar manner to that of Example l.
mp : 275-277°C
IR (Nujol) : l580, l480 cm⁻¹
NMR (DMSO-d₆, δ) : l.58-l.85 (2H, m), 2.l2 (3H, s), 2.38-3.25 (4H, m), 7.20-7.55 (9H, m)
Mass (M/Z) : 236 (M⁺ - l)

| Elemental Analysis for C₁₇H₁₉N^{.}HCl^{.}l/5H₂O | | | |
|---|---|---|---|
| Calcd. | C 73.60, | H 7.4l, | N 5.04 |
| Found | C 74.05, | H 7.82, | N 5.05 |

The following compounds (Examples 33 to 37) were obtained according to a similar manner to that of Example 28.

### Example 33

l-Methyl-l-phenyl-2-ethyl-l,2,3,4-tetrahydroisoquinoline hydrochloride
IR (Nujol) : l490, l420, l400 cm⁻¹

### Example 34

l,2-Dimethyl-l-(2-thienyl)-l,2,3,4-tetrahydroisoquinoline hydrochloride
IR (Nujol) : 2320, l260, 725 cm⁻¹

### Example 35

l-Methyl-l-(2-thienyl)-2-ethyl-l,2,3,4-tetrahydroisoquinoline hydrochloride
IR (Nujol) : ll75, 740 cm⁻¹

### Example 36

l-Methyl-l-phenyl-2-allyl-l,2,3,4-tetrahydroisoquinoline
IR (Film) : l640, l600, l490, l450 cm⁻¹

### Example 37

l-Methyl-l-phenyl-2-phenethyl-l,2,3,4-tetrahydroisoquinoline hydrochloride
IR (Nujol) : l600, l580, l490, l420 cm⁻¹ The following compounds (Examples 38 and 39) were obtained according to a similar manner to that of Example 22.

### Example 38

l-Methyl-l-phenyl-2-(2-piperidinoacetyl)-l,2,3,4-tetrahydroisoquinoline hydrochloride
IR (Nujol) : 3340, l660 cm⁻¹

### Example 39

l-Methyl-l-phenyl-2-(2-piperidinoethyl)-l,2,3,4-tetrahydroisoquinoline dihydrochloride
IR (Nujol) : 3380, l590 cm⁻¹

### Example 40

l-Methyl-l-phenyl-2-(2-piperidinoacetyl)-l,2,3,4-tetrahydroisoquinoline hydrochloride was obtained according to a similar manner to that of Example 29.
IR (Nujol) : 3340, l660 cm⁻¹

### Example 4l

l-Methyl-l-phenyl-2-(2-piperidinoethyl)-l,2,3,4-tetrahydroisoquinoline dihydrochloride was obtained according to a similar manner to that of Example 28.
IR (Nujol) : 3380, l590 cm⁻¹

## Claims

1. A bicyclic amine compound of the formula : 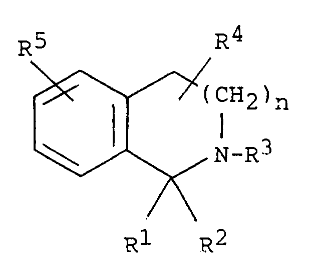 wherein
R¹ is (C₁-C₆)alkyl,
R² is phenyl which may have 1 to 3 suitable substituent(s) selected from a group consisting of (C₁-C₆)alkyl, (C₁-C₆)alkoxy and halogen; cyclo(C₃-C₆)alkyl, furyl, or thienyl,
R³ is hydrogen, (C₁-C₆)alkyl which may have 1 to 3 suitable substituent(s) selected from a group consisting of phenyl and piperidino, (C₂-C₆)alkenyl, or (C₁-C₆)alkanoyl which may have 1 to 3 suitable substituent(s) selected from a group consisting of halogen and piperidino,
R⁴ is hydrogen, (C₁-C₆)alkyl, or hydroxy(C₁-C₆)alkyl,
R⁵ is hydrogen, (C₁-C₆)alkyl, halogen, or (C₁-C₆)alkoxycarbonylamino, and
n is an integer of 1 or 2,
with proviso that R¹ is not ethyl, when R³ is methyl and n is 1.
and pharmaceutically acceptable salt thereof.

2. A compound of claim 1, wherein
R² is phenyl, phenyl having (C₁-C₆)alkyl, phenyl having (C₁-C₆)alkoxy, phenyl having halogen, cyclo(C₃-C₆)alkyl, furyl, or thienyl,
R³ is hydrogen, (C₁-C₆)alkyl, (C₁-C₆)alkyl having phenyl, (C₁-C₆)alkyl having piperidino, (C₂-C₆)alkenyl, (C₁-C₆)alkanoyl having halogen, or (C₁-C₆)alkanoyl having piperidino,
R⁵ is hydrogen, (C₁-C₆)alkyl, halogen, or (C₁-C₆)alkoxycarbonylamino, and
n is an integer of 1.

3. A compound of claim 2, wherein
R¹ is methyl,
R² is phenyl, furyl, or thienyl, and
R³, R⁴ and R⁵ are each hydrogen.

4. A compound of claim 3, which is 1-methyl-1-phenyl-1,2,3,4-tetrahydroisoquinoline or its hydrochloride.

5. A process for preparing a bicyclic amine of the formula : 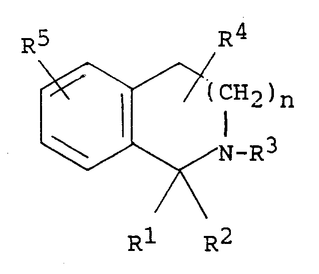 wherein
R¹ is (C₁-C₆)alkyl,
R² is phenyl which may have 1 to 3 suitable substituent(s) selected from a group consisting of (C₁-C₆)alkyl, (C₁-C₆)alkoxy and halogen; cyclo(C₃-C₆)alkyl, furyl, or thienyl,
R³ is hydrogen,
R⁴ is hydrogen, (C₁-C₆)alkyl, or hydroxy(C₁-C₆)alkyl,
R⁵ is hydrogen, (C₁-C₆)alkyl, halogen, or (C₁-C₆)alkoxycarbonylamino,
or a salt thereof, which comprises
subjecting a compound of the formula : 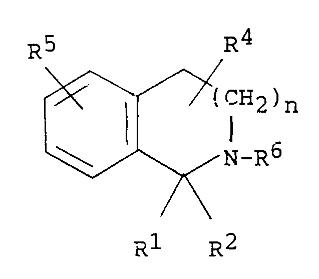 wherein
R¹, R², R⁴, R⁵ and n are each as defined above, and
R⁶ is phenyl(C₁-C₆)alkyl which may have (C₁-C₆)alkoxy,
or a salt thereof,
to elimination reaction of R⁶ to give a compound of the formula : 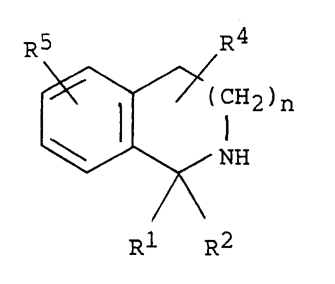 wherein
R¹, R², R⁴, R⁵ and n are each as defined above,
or a salt thereof.

6. A compound of the formula : 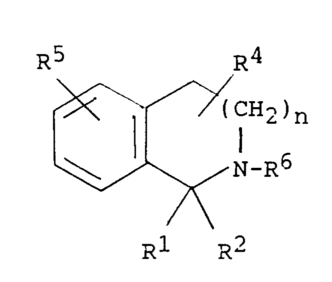 wherein
R¹, R², R⁴, R⁵ and n are each as defined above, and
R⁶ is phenyl(C₁-C₆)alkyl which may have (C₁-C₆)alkoxy,
or a salt thereof.

7. A pharmaceutical composition comprising a compound of claim 1, as an active ingredient, in association with a pharmaceutically acceptable, substantially nontoxic carrier or excipient.

8. A compound of claim 1 for use as a medicament.

9. Use of a compound of claim 1 for the manufacture of a medicament for the therapeutic treatment of convulsion and delayed neuronal death.

## Patentansprüche

1. Bicyclische Aminverbindung der Formel: 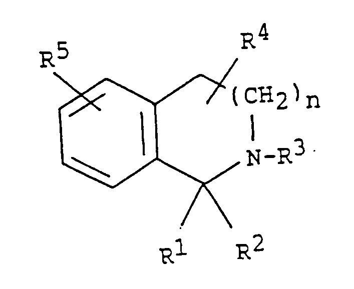 worin
R¹ (C₁-C₆)Alkyl ist,
R² Phenyl, das 1 bis 3 geeignete(n) Substituent(en), ausgewählt aus der Gruppe, bestehend aus (C₁-C₆)Alkyl, (C₁-C₆)Alkoxy und Halogen, aufweisen kann; Cyclo(C₃-C₆)alkyl, Furyl, oder Thienyl ist,
R³ Wasserstoff, (C₁-C₆)Alkyl, das einen bis drei geeignete(n) Substituent(en), ausgewählt aus der Gruppe, bestehend aus Phenyl und Piperidino, aufweisen kann, (C₂-C₆)Alkenyl oder (C₁-C₆)Alkanoyl ist, das 1 bis 3 geeignete(n) Substituent(en), ausgewählt aus der Gruppe, bestehend aus Halogen und Piperidino, aufweisen kann,
R⁴ Wasserstoff, (C₁-C₆)Alkyl oder Hydroxy(C₁-C₆)alkyl ist,
R⁵ Wasserstoff, (C₁-C₆)Alkyl, Halogen oder (C₁-C₆)Alkoxycarbonylamino ist, und
n eine ganze Zahl von 1 oder 2 ist,
mit der Maßgabe, daß R¹ nicht Ethyl ist, wenn R³ Methyl und n 1 ist,
und pharmazeutisch verträgliche Salze davon.

2. Verbindung nach Anspruch 1, worin
R² Phenyl, Phenyl mit (C₁-C₆)Alkyl, Phenyl mit (C₁-C₆)Alkoxy, Phenyl mit Halogen, Cyclo(C₃-C₆)alkyl, Furyl oder Thienyl ist,
R³ Wasserstoff, (C₁-C₆)Alkyl, (C₁-C₆)Alkyl mit Phenyl, (C₁-C₆)Alkyl mit Piperidino, (C₂-C₆)Alkenyl, (C₁-C₆)Alkanoyl mit Halogen oder (C₁-C₆)Alkanoyl mit Piperidino ist,
R⁵ Wasserstoff, (C₁-C₆)Alkyl, Halogen oder (C₁-C₆)Alkoxycarbonylamino ist, und
n ist eine ganze Zahl von 1.

3. Verbindung nach Anspruch 2, worin
R¹ Methyl ist,
R² Phenyl, Furyl oder Thienyl ist, und
R³, R⁴ und R⁵ jeweils Wasserstoff sind.

4. Verbindung nach Anspruch 3, die 1-Methyl-1-phenyl-1,2,3,4-tetrahydroisochinolin oder ihr Hydrochlorid ist.

5. Verfahren zur Herstellung eines bicyclischen Amins der Formel 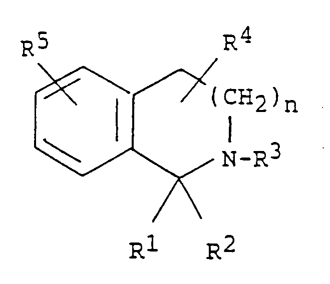 worin
R¹ (C₁-C₆) Alkyl ist,
R² Phenyl, das 1 bis 3 geeignete(n) Substituent(en), ausgewählt aus der Gruppe, bestehend aus (C₁-C₆)Alkyl, (C₁-C₆)Alkoxy und Halogen, aufweisen kann; Cyclo(C₃-C₆)alkyl, Furyl oder Thienyl ist,
R³ Wasserstoff ist, R⁴ Wasserstoff, (C₁-C₆)Alkyl oder Hydroxy(C₁-C₆)alkyl ist, R⁵ Wasserstoff, (C₁-C₆)Alkyl, Halogen oder (C₁-C₆) Alkoxycarbonylamino ist,
oder eines Salzes davon, das umfaßt:
Unterwerfen einer Verbindung der Formel: 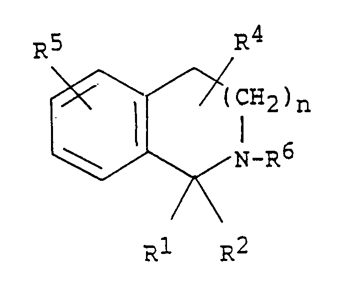 worin
R¹, R², R⁴, R⁵ und n wie oben definiert sind, und
R⁶ Phenyl(C₁-C₆)alkyl ist, das (C₁-C₆)Alkoxy aufweisen kann,
oder eines Salzes davon der Eliminierungsreaktion von R⁶, um
eine Verbindung der Formel: 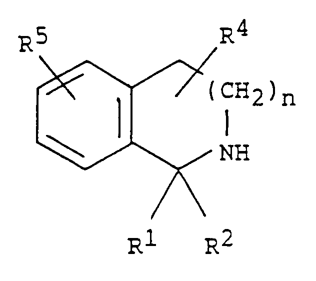 worin
R¹, R², R⁴, R⁵ und n wie oben definiert sind, oder ein Salz davon zu ergeben.

6. Verbindung der Formel: 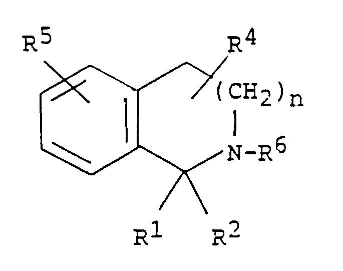 worin
R¹, R², R⁴, R⁵ und n wie oben definiert sind, und
R⁶ Phenyl(C₁-C₆)alkyl ist, das (C₁-C₆) Alkoxy aufweisen kann,
oder ein Salz davon.

7. Pharmazeutische Zusammensetzung, die eine Verbindung nach Anspruch 1 als aktiven Bestandteil in Assoziation mit einem pharmazeutisch verträglichen, im wesentlichen nicht giftigen Träger oder Exzipienten umfaßt.

8. Verbindung nach Anspruch 1 zur Verwendung als Medikament.

9. Verwendung einer Verbindung nach Anspruch 1 zur Herstellung eines Medikamentes für die therapeutische Behandlung von Konvulsion und verzögertem neuronalen Tod.

## Revendications

1. Composé d'amine bicyclique répondant à la formule : 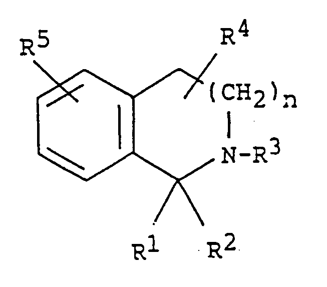 dans laquelle
R¹ représente un groupe alkyle en (C₁-C₆),
R² représente un groupe phényle qui peut avoir 1 à 3 substituant(s) approprié(s) choisi(s) dans le groupe constitué par un groupe alkyle en (C₁-C₆), un groupe alcoxy en (C₁-C₆) et un atome d'halogène;
un groupe cycloalkyle en (C₃-C₆), un groupe furyle, ou un groupe thiényle,
R³ représente un atome d'hydrogène, un groupe alkyle en (C₁-C₆) qui peut avoir 1 à 3 substituant(s) approprié(s) choisi(s) dans le groupe constitué du groupe phényle et du groupe pipéridino, un groupe alcényle en (C₂-C₆), ou un groupe alcanoyle en (C₁-C₆) qui peut avoir 1 à 3 substituant(s) approprié(s) choisi(s) dans le groupe constitué d'un atome d'halogène et du groupe pipéridino,
R⁴ représente un atome d'hydrogène, un groupe alkyle en (C₁-C₆), ou un groupe hydroxyalkyle en (C₁-C₆),
R⁵ représente un atome d'hydrogène, un groupe alkyle en (C₁-C₆), un atome d'halogène ou un groupe alcoxy (en C₁-C₆)carbonylamino et
n représente un nombre entier égal à 1 ou 2,
à condition que R¹ ne représente pas un groupe éthyle lorsque R³ représente un groupe méthyle et que n est égal à 1,
et un sel pharmaceutiquement acceptable de ce composé.

2. Composé selon la revendication 1, dans lequel :
R² représente un groupe phényle, un groupe phényle portant un groupe alkyle en (C₁-C₆), un groupe phényle portant un groupe alcoxy en (C₁-C₆), un groupe phényle portant un atome d'halogène, un groupe cycloalkyle en (C₃-C₆), un groupe furyle ou un groupe thiényle,
R³ représente un atome d'hydrogène, un groupe alkyle en (C₁-C₆), un groupe alkyle en (C₁-C₆) portant un groupe phényle, un groupe alkyle en (C₁-C₆) portant un groupe pipéridino, un groupe alcényle en (C₂-C₆), un groupe alcanoyle en (C₁-C₆) portant un atome d'halogène, ou un groupe alcanoyle en (C₁-C₆) portant un groupe pipéridino,
R⁵ représente un atome d'hydrogène, un groupe alkyle en (C₁-C₆), un atome d'halogène, ou un groupe alcoxy (en C₁-C₆)carbonylamino, et
n représente un nombre entier égal à 1.

3. Composé selon la revendication 2, dans lequel :
R¹ représente un groupe méthyle,
R² représente le groupe phényle, le groupe furyle ou le groupe thiényle, et
R³, R⁴ et R⁵ représentent chacun un atome d'hydrogène.

4. Composé selon la revendication 3, qui est la 1-méthyl-1-phényl-1,2,3,4-tétrahydroisoquinoléine ou son chlorhydrate.

5. Procédé de préparation d'une amine bicyclique répondant à la formule : 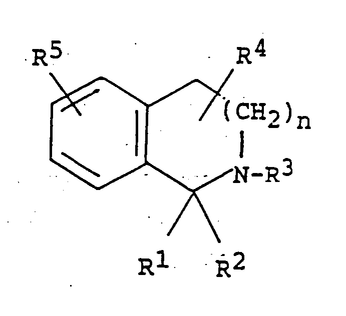 dans laquelle
R¹ représente un groupe alkyle en (C₁-C₆),
R² représente un groupe phényle qui peut avoir 1 à 3 substituant(s) approprié(s) choisi(s) dans le groupe constitué par un groupe alkyle en (C₁-C₆), un groupe alcoxy en (C₁-C₆) et un atome d'halogène;
un groupe cycloalkyle en (C₃-C₆), un groupe furyle, ou un groupe thiényle,
R³ représente un atome d'hydrogène,
R⁴ représente un atome d'hydrogène, un groupe alkyle en (C₁-C₆), ou un groupe hydroxyalkyle en (C₁-C₆),
R⁵ représente un atome d'hydrogène, un groupe alkyle en (C₁-C₆), un atome d'halogène ou un groupe alcoxy (en C₁-C₆)carbonylamino,
ou un sel de ce composé, qui consiste à faire subir à un composé répondant à la formule : 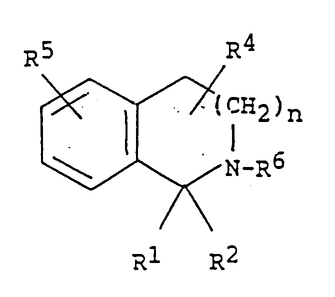 dans laquelle
R¹, R², R⁴, R⁵ et n sont chacun tels que définis ci-dessus, et R⁶ représente un groupe phényl(alkyle en (C₁-C₆)) qui peut porter un groupe alcoxy en (C₁-C₆),
ou un sel de ce composé,
à une réaction d'élimination de R⁶ pour obtenir un composé répondant à la formule : 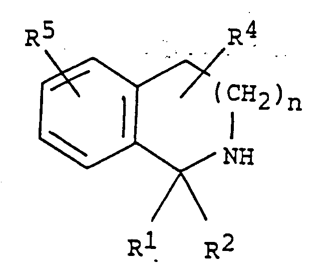 dans laquelle
R¹, R², R⁴, R⁵ et n sont chacun tels que définis ci-dessus,
ou un sel de ce composé.

6. Composé répondant à la formule : 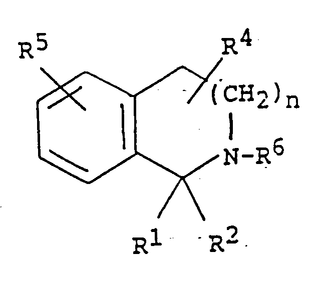 dans laquelle
R¹, R², R⁴, R⁵ et n sont chacun tels que définis ci-dessus, et
R⁶ représente un groupe phényl(alkyle en (C₁-C₆)) qui peut porter un groupe alcoxy en (C₁-C₆),
ou un sel de ce composé,

7. Composition pharmaceutique comprenant un composé selon la revendication 1, comme constituant actif, en association avec un véhicule ou un excipient pharmaceutiquement acceptable, essentiellement non-toxique.

8. Composé selon la revendication 1, destiné à être utilisé comme médicament.

9. Utilisation d'un composé selon la revendication 1, pour la fabrication d'un médicament pour le traitement thérapeutique des convulsions et de la mort neuronale retardée.
